# EUROPEAN PATENT APPLICATION

(11) **EP 3 424 520 A1**
(43) Date of publication of application: **09.01.2019**
(21) Application number: 16877837.1
(22) Date of filing: 21.12.2016
(51) Int. Cl.: A61K 38/17, C07K 14/715, A61P 31/12

(54) **METHOD FOR OBTAINING IFNAR RECOMBINANT PROTEIN, AND USE THEREOF AS AN ANTIVIRAL**

(30) Priority: 21.12.2015 ES 201531862
(71) Applicant: Servicio Andaluz de Salud, 41071 Sevilla (ES); Universidad de Málaga, 29071 Málaga (ES)
(72) Inventor: FERNÁNDEZ FERNÁNDEZ,Óscar, 41071 Sevilla (ES); OLIVER MARTOS, Begoña, 41071 Sevilla (ES); ÓRPEZ ZAFRA, Teresa, 41071 Sevilla (ES); LEYVA FERNÁNDEZ, Laura, 41071 Sevilla (ES); PAVÍA MOLINA, José, 29071 Málaga (ES)
(74) Representative: Hoffmann Eitle
(86) International application number: PCT/ES2016/070917
(87) International publication number: WO 2017/109257

(57) **Abstract**

The present invention relates to a method for obtaining IFNAR recombinant protein and to the use thereof in the preparation of a medicinal product for preventing, controlling, treating, and/or alleviating a viral disease.

## Description

### FIELD OF THE INVENTION

The present invention is comprised in the field of biomedicine and biotechnology and relates to the use of the isolated soluble IFNAR2.3 receptor produced in a recombinant manner in the prevention, control, treatment, and/or alleviation of a viral disease.

### BACKGROUND OF THE INVENTION

The progress made in viral disease therapy has not been as significant as the progress achieved for the treatment of bacterial infections. The development of highly effective broad-spectrum antiviral agents is a primary objective shared by the fields of virology and pharmacology.

Viruses are intracellular parasites which use the metabolic machinery of the infected host cell. Therefore, the development of antivirals presents a series of difficulties associated with said obligate parasitic character. Achieving suitable antiviral activity without affecting host cell metabolism and without causing negative effects in other uninfected cells of the body is an uphill task.

Current strategies for controlling viral infectivity are based on identifying agents capable of intervening in the essential stages of viral infection, such as the entry (fusion, endocytosis), replication, assembly, in addition to drugs targeting the viral envelope. Another strategy would target the modulation of the cell defense system.

The identification of broad-spectrum antiviral agents focusing on reducing viral infectivity or modulating host defenses would constitute a significant contribution in the field of virology for improving human health and controlling viral outbreaks.

IFNβ carries out its biological activity through interaction with the surface receptor IFNAR that is formed by two subunits, IFNAR1 and IFNAR2. Dimerization of the two subunits and activation of the intracellular signaling cascade, the signal of which is transduced to the nucleus through the Jak-Stat pathway, take place upon the binding of IFNβ to IFNAR2. The antiviral activities, antiproliferative activities, and immunomodulatory activities of IFNβ are carried out in this manner.

The IFNAR2 subunit of the receptor undergoes alternative mRNA processing which gives rise to three different isoforms: a short isoform (IFNAR2b), a functionally active long isoform (IFNAR2c), and the soluble isoform (sIFNAR2, IFNAR2.3, or IFNAR2a). Only IFNAR2c acts as a functional receptor together with IFNAR1 and is capable of mediating the biological effects of IFNβ. sIFNAR2, which lacks cytoplasmic and transmembrana domains, has been identified in human biological fluids, and even though its role is not defined, it has been suggested that sIFNAR2 may exhibit the capacity to neutralize the binding of IFNβ to the IFNAR2 receptor. In this manner, it may perform modulatory functions according to its concentration, where it may, on one hand, neutralize the binding of IFNβ to the IFNAR receptor, or otherwise, prolong the half-life of the circulating IFNβ, preventing its degradation or the formation of oligomers. The function of the soluble IFNAR2 variant remains unknown to this day.

The inventors of the present invention have confirmed the antiviral effect of an sIFNAR2 recombinant protein, similar to the soluble isoform of the IFNβ receptor.

### BRIEF DESCRIPTION OF THE INVENTION

A first aspect of the invention relates to the use of a recombinant protein, hereinafter recombinant protein of the invention, obtainable by means of a method which comprises:
a) integrating an insert with the nucleotide sequence SEQ ID NO: 1 in a gene construct or an expression vector,
b) transforming a host with the expression vector of step (a),
c) inducing expression of the recombinant protein,
d) extracting the recombinant protein, and
e) purifying the recombinant protein,
in the preparation of a medicinal product for preventing, improving, treating, and/or alleviating a viral disease.

In a preferred embodiment of this aspect of the invention, the expression vector is the prelinearized vector pEcoli-Cterm 6xHN Linear.

In another preferred embodiment of this aspect of the invention, the host of step (b) is expression bacteria. The expression bacteria are preferably bacteria *E.coli* BL21(DE).

In another preferred embodiment of this aspect of the invention, the integration of the nucleotide sequence SEQ ID NO: 1 of step (a) is performed by means of a ligation process.

In another preferred embodiment of the invention, a lyophilisate comprising the In-Fusion enzyme is used in the ligation.

In another preferred embodiment, the insert was synthesized using primers with the nucleotide sequence SEQ ID NO: 3 and SEQ ID NO: 4.

Another aspect of the invention relates to the use of an antibody or a fragment thereof, hereinafter antibody of the invention, which specifically recognizes the recombinant protein of the invention, in the preparation of a medicinal product for preventing, controlling, treating, and/or alleviating a viral disease.

In a preferred embodiment of this aspect of the invention, where the antibody is obtained by means of injecting the recombinant protein of the invention in a suitable animal, and collecting, and optionally by means of purifying, the antisera of the animals.

In another preferred embodiment of this aspect of the invention, the antibody is a monoclonal antibody.

Another aspect of the invention relates to the use of a composition, hereinafter composition of the invention, comprising:
a) a protein comprising the amino acid sequence SEQ ID NO: 2,
b) the recombinant protein of the invention,
c) the protein of the invention (soluble IFNAR2, sIFNAR2, or IFNAR2.3) obtained by other non-recombinant means, and/or
d) the antibody, or a fragment thereof, of the invention,
in the preparation of a medicinal product for preventing, improving, treating, and/or alleviating a viral disease.

In a preferred embodiment of this aspect of the invention, the composition is a pharmaceutical composition which optionally further comprises a pharmaceutically acceptable vehicle and/or pharmaceutically acceptable excipients.

In another preferred embodiment of this aspect of the invention, the composition of the invention further comprises another active ingredient. This active ingredient is preferably selected from the list consisting of other antivirals, analgesics, antipyretics, decongestants, or other active ingredients used in the treatment of viral diseases.

In another preferred embodiment of this aspect of the invention, the viral disease is caused by a virus which is selected from the list consisting of: hepatitis A virus (HAV), hepatitis B virus (HBV), hepatitis C virus (HCV), hepatitis D virus (HDV), hepatitis E virus (HEV), hepatitis F virus, or hepatitis G virus (HGV), human immunodeficiency virus (HIV), respiratory syncytial virus, influenza virus, encephalomyocarditis virus, vesicular stomatitis virus, or any combinations thereof.

### DESCRIPTION OF THE DRAWINGS

**Figure 1****.** Representative bioassay conducted in the presence of IFNAR2.3. The more intense violet color corresponds with greater cell survival after infecting the cell culture with the virus.

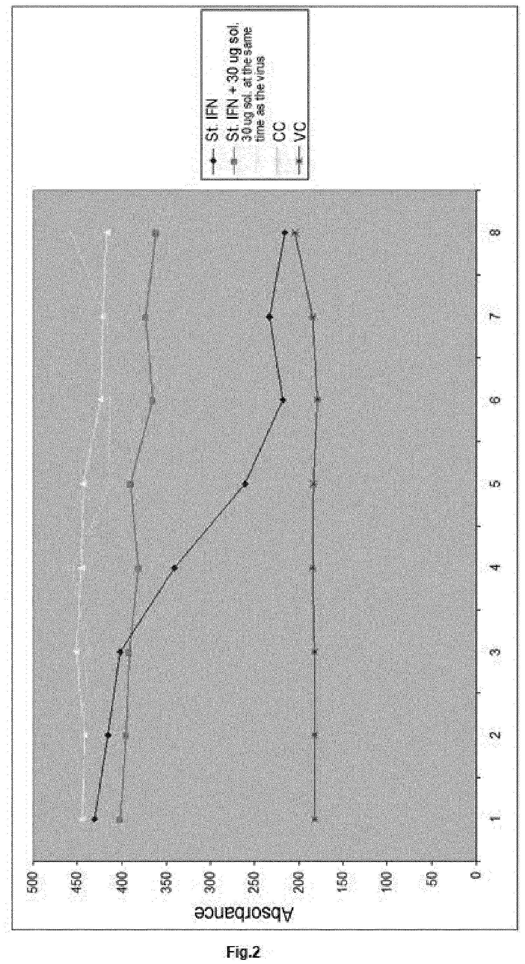

**Figure 2****.** Depiction of the absorbance values obtained in the preceding bioassay under each of the conditions. The manner in which IFNAR2.3 protect the cells from the action of the virus can be seen, the absorbance values being very close to the cell control and well above the viral control.
**Figure 3****.** Bar graph depicting the absorbance values obtained with the three tested concentrations of sIFNAR2. The results represent the mean of the 8 replicates which have been performed for each of the concentrations. The manner in which sIFNAR2 protects the cells from the action of the virus can be seen, the absorbance values being well above the viral control in a statistically significant manner (p = 0.012 in all the comparisons).
**Figure 4****.** Second bioassay dish for a bioassay conducted in the presence of sIFNAR2, showing its antiviral capacity. Furthermore, the bioassay has been conducted in the presence of B18R, an IFNβ inhibitor. It is demonstrated that B18R blocks the action of IFNβ but not the action of the sIFNAR2 recombinant protein; the manner in which the cells remain protected in the presence of B18R+sIFNAR2 can be seen.

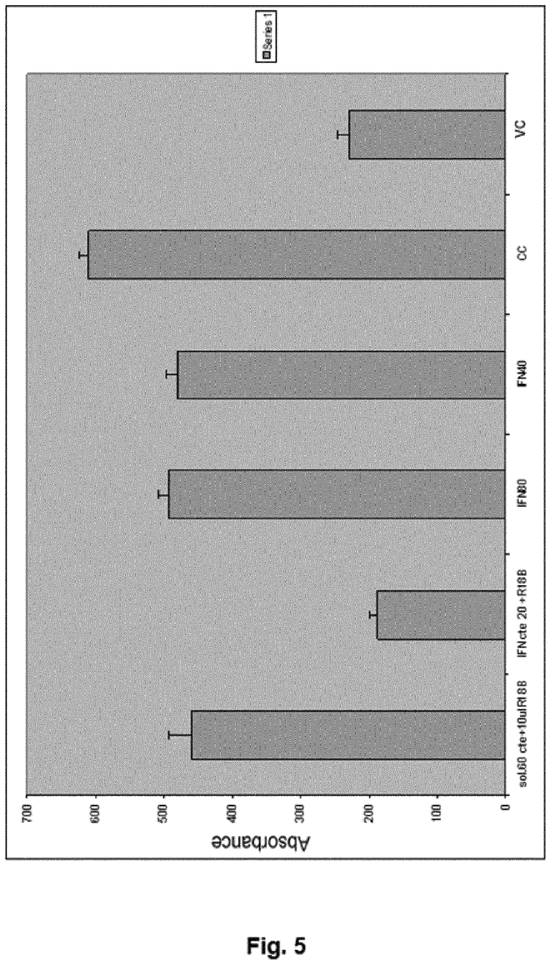

**Figure 5****.** Bar graph depicting the absorbances obtained in the bioassay of Figure 4, of the viral control (VC), cell control (CC), IFN40, and IFN80. Furthermore, the absorbances obtained for IFNβ and sIFNAR2 in the presence of B18R are depicted. The manner in which B18R inhibits the action of IFNβ can be seen and the cells are not protected. However, B18R does not inhibit the antiviral capacity of recombinant sIFNAR2, the absorbance being above the VC.

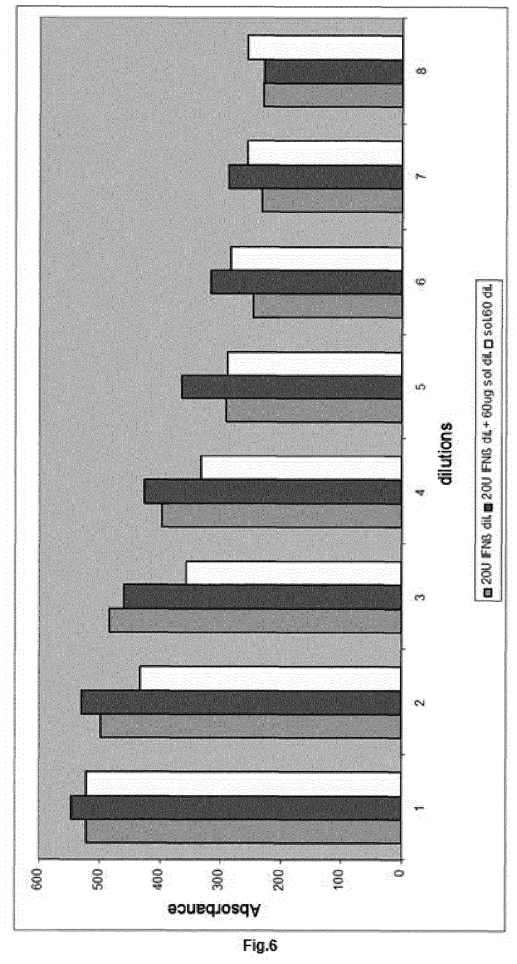

**Figure 6****.** Graph depicting the absorbances of the bioassay of Figure 4 as a function of the different dilutions obtained in the presence of IFNβ alone, the combination of IFNβ+sIFNAR2, and sIFNAR2 *per se.* It is shown that recombinant sIFNAR2 protects the cell culture in the same way as IFNβ. On the other hand, the manner in which the combination of both molecules provides additional protection to the cell culture in most of the dilutions can be seen, a higher absorbance being obtained. sIFNAR2 seems to enhance the effect of IFNβ and vice versa.

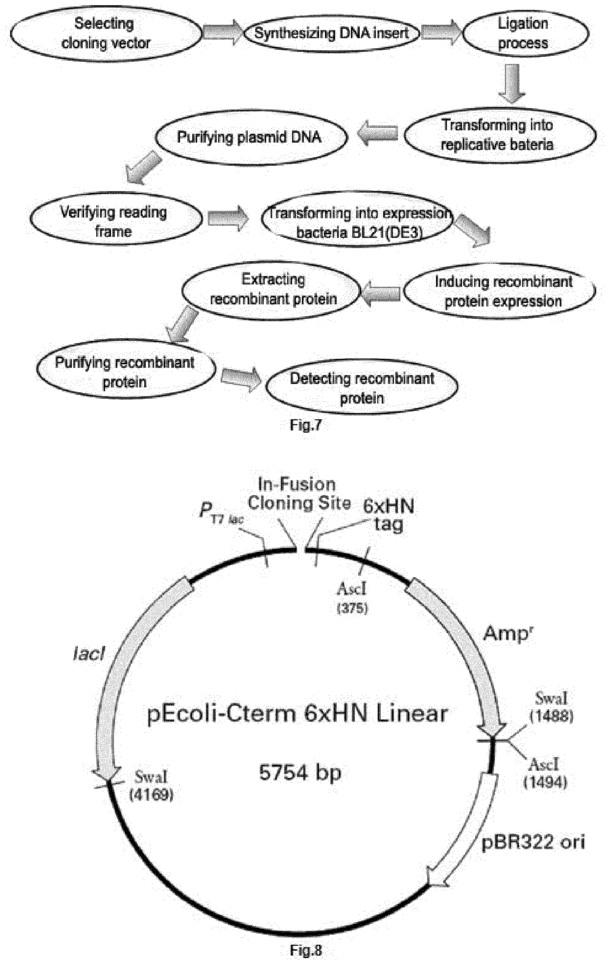

**Figure 7****.** Work diagram illustrating the cloning, production, and purification of the recombinant protein.
**Figure 8****.** Structure of the vector pEcoli-Cterm 6xHN Linear.

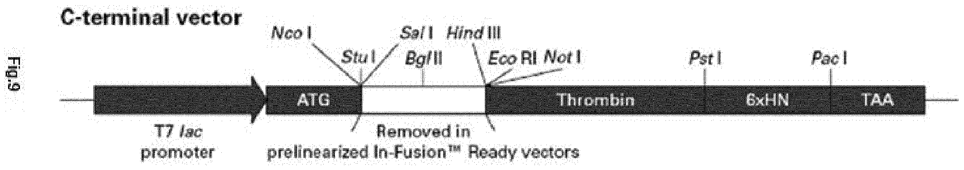

**Figure 9****.** Structure of the structures flanking the insert.

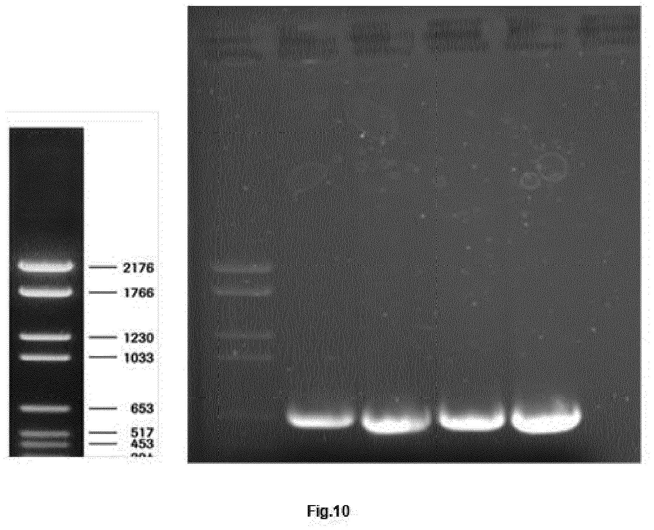

**Figure 10****.** Agarose gel electrophoresis of the amplification product obtained by means of PCR.

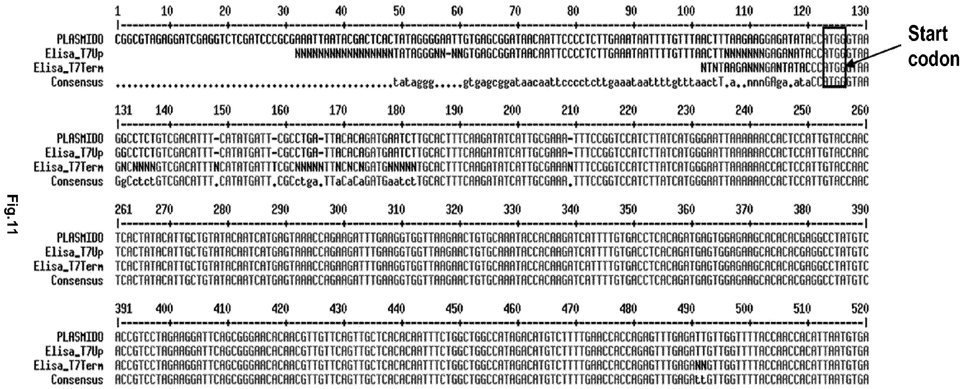

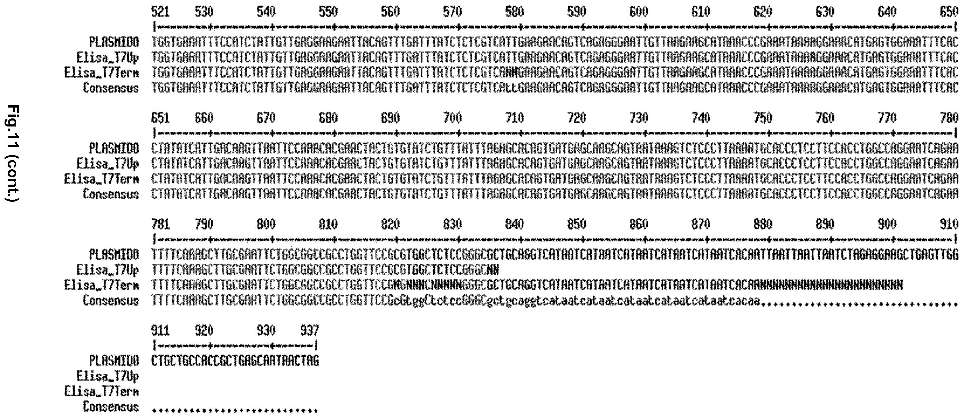

**Figure 11****.** Alignments of the nucleotide sequences in 5'-3' direction. The first line shows the nucleotide sequence of IFNAR2.3, the second and third lines show the nucleotide sequences with the primers flanking the insert T7UP and T7terminal, obtained after the plasmid sequencing process.

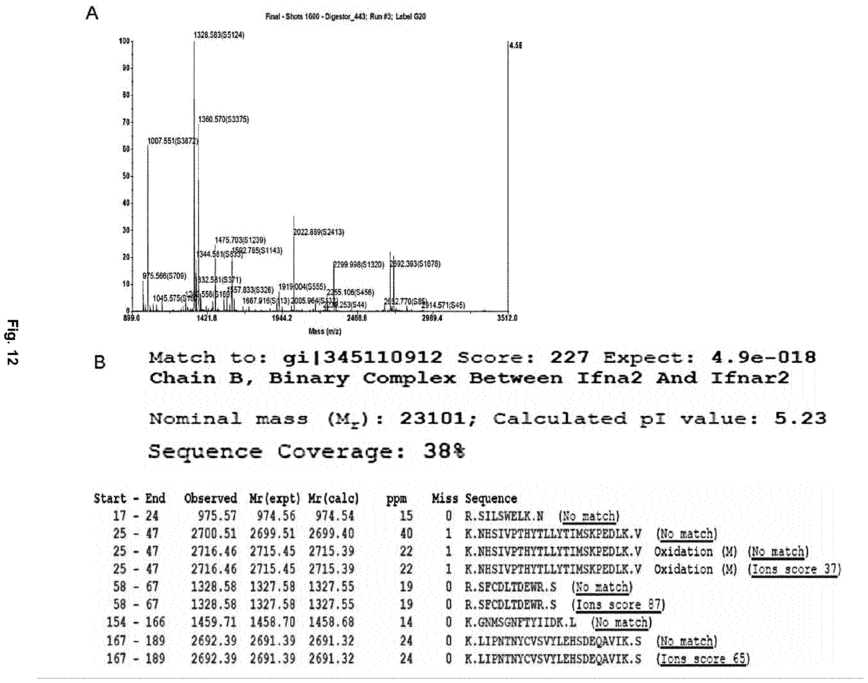

**Figure 12****.** Identification of the sIFNAR2 protein by means of MALDI-OF.
**Figure 13****.** An A549/encephalomyocarditis virus bioassay with different batches of sIFNAR2 protein as described in Example 3.
**Figure 14****.** Results of a bioassay performed with the HEP2 cell line and vesicular stomatitis virus at Centro Severo Ochoa, CSIC, Madrid. Cell viability is determined with MTT. The dish included a non-infected cell control, a standard IFNβ (100, 20, 10, 5 IU), and a viral control (without IFNβ). Furthermore, two concentrations of recombinant sIFNAR2 were included, added on the second and third days of the assay.
**Figure 15****.** Post-infection results of, on one hand, macrophages differentiated from peripheral blood monocytes by adherence under the conditions established in Example 5.
**Figure 16****.** Peripheral blood lymphocytes activated with PHA+IL2 were infected with a luciferase-expressing viral vector. The expression of luciferase is therefore measured under the control of the promoter and regulation of HIV itself. A decrease in infection of about 50% is observed at 48 hours of infection.

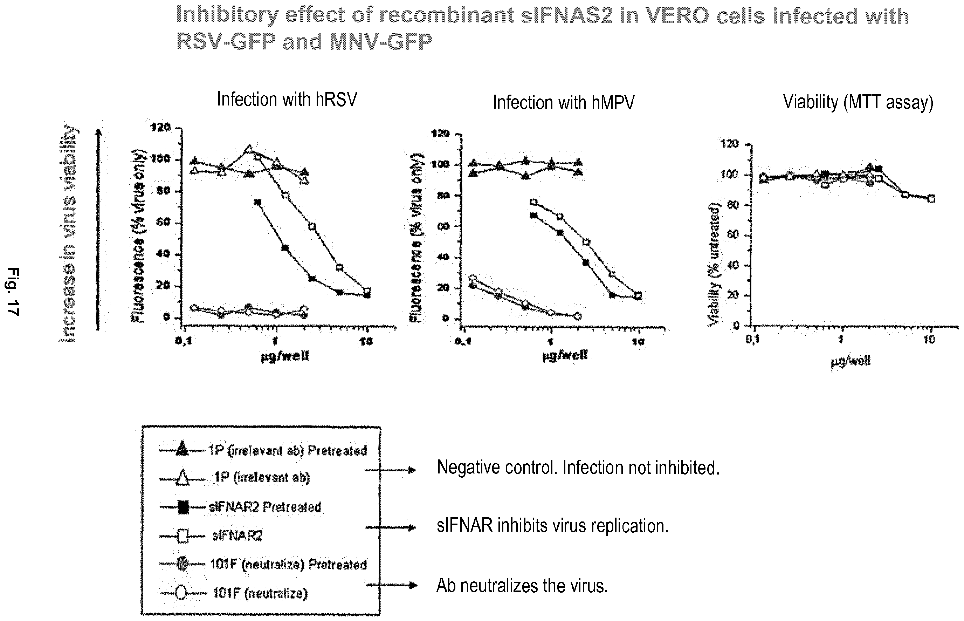

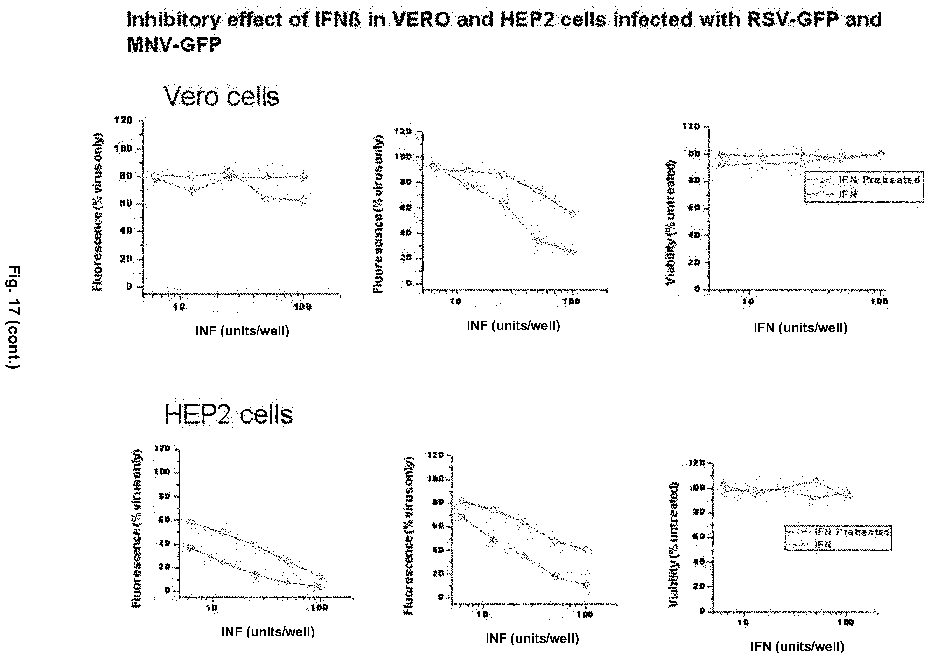

**Figure 17****.** Vero cells were treated with the indicated amounts of sIFNAR2, IFNβ, or the indicated mABs. 101F is a monoclonal antibody (mAb) which neutralizes human respiratory syncytial virus (hRSV) and human metaneumovirus (hMPV), and 1P is an irrelevant antibody (negative control). After 48 hours, the cells were infected with a fixed amount of GFP-expressing recombinant viruses (hRSV-GFP or hMNV-GFP) in the presence of sIFNAR2, IFNβ, or mABs. The same amount of each of these reagents was again added to the pretreated cultures at the time of infection. Furthermore, an MTT viability assay was performed at 48 hours in parallel in non-infected Vero cell cultures that were, however, treated with sIFNAR2, IFNβ, or mABs.

### DETAILED DESCRIPTION OF THE INVENTION

### USE OF THE RECOMBINANT PROTEIN OF THE INVENTION

The authors of the present invention have studied the effects of using an sIFNAR2 recombinant protein in the prevention, control, treatment, and/or alleviation of a viral disease.

Specifically, cloned and purified sIFNAR2 protein was used. Furthermore, a histidine-asparagine tag was added to the carboxy terminal end by means of the cloning method used, being fused to the recombinant protein as a label. After the production of the recombinant protein in the host cell, the cell lysate is passed through an affinity column for purification. The fusion protein with the label is retained in the column while other proteins and contaminants flow through said column.

Therefore, a first aspect of the invention relates to the use of a recombinant protein, hereinafter recombinant protein of the invention, obtainable by means of a method which comprises:
a) integrating an insert with the nucleotide sequence SEQ ID NO: 1 in a gene construct or an expression vector,
b) transforming a host with the expression vector of step (a),
c) inducing expression of the recombinant protein,
d) extracting the recombinant protein, and
e) purifying the recombinant protein,
in the preparation of a medicinal product for preventing, controlling, treating, and/or alleviating a viral disease.

The design of the vector based on genetic engineering techniques and the selection of the host cell determine to a large extent the characteristics of the recombinant protein.

The gene construct of a) can comprise, in addition to the nucleotide sequence SEQ ID NO: 1, elements regulating the expression of said sequence. Said regulatory elements include promoters and enhancers. Promoters are typically positioned in the 5' position with respect to the transcription or translation start site. Enhancers are capable of influencing the expression of genes when they are in the 5' or 3' position with respect to the cDNA or when they are part of an intron. Regulatory sequences include, in addition to promoters, sequences which facilitate translation, processing signals for introns, end codons, signal sequences, internal ribosome-binding sites (IRES), and polyadenylation signals.

The expression vector of a) comprising the nucleotide sequence SEQ ID NO: 1 or the gene construct of a) is operatively coupled with a sequence regulating the expression of said nucleotide sequence SEQ ID NO: 1 or said gene construct. One skilled in the art will know that the type of vector suitable for expression of the nucleic acids and gene constructs of the invention will depend on the organism in which the polynucleotide of the invention is to be expressed.

In a preferred embodiment of this aspect of the invention, the expression vector is the prelinearized vector pEcoli-Cterm 6xHN Linear.

In another preferred embodiment of this aspect of the invention, the protein is cloned into baculovirus and/or the vectors used are selected, without limitation, from the list consisting of: pAcP(+)IE1-1, pAcP(+)IE1-2, pAcP(+)IE1-3, pAcP(+)IE1-4, pAcP(+)IE1-5, pAcP(+)IE1-6, pAcUW31, pBAC-1, pBAC-2cp, pBAC-3, pBAC4x-1, pBAC-7, pBAC-8, pBAC-9, pBAC-10, pBacPAK8, pBacPAK9, pBACsurf-1, pBlueBac4.5, pBlueBacHis2, pFastBac1, pFastBac HT, pFastBac DUAL, pMbac, pMelBac, pPbac, pTriEx-1, pVL1392, and pVL1393. The promoters used in these vectors are preferably: the ie1 promoter in the case of pAcP(+)IE1-1, pAcP(+)IE1-2, pAcP(+)IE1-3, pAcP(+)IE1-4, pAcP(+)IE1-5, pAcP(+)IE1-6; the polh promoter in the case of pAcUW31, pBAC-1, pBAC-2cp, pBAC-3, pBAC4x-1, pBAC-7, pBAC-8, pBAC-9, pBAC-10, pBacPAK8, pBacPAK9, pBACsurf-1, pBlueBac4.5, pBlueBacHis2, pFastBac1, pFastBac HT, pFastBac DUAL, pMbac, pMelBac, pPbac, pVL1392, and pVL1393; the p10 promoter pAcUW31, pBAC4x-1, pFastBac DUAL, pMbac, pMelBac, pPbac and pTriEx-1. The clones are preferably selected by means of antibiotic-resistance genes introduced in the vector, more preferably ampicillin- and/or gentamicin-resistance genes.

A host cell or organism can comprise the gene construct or a vector as defined in the invention. In principle, any type of host organism known to one skilled in the art can be used in the present invention, such as a bacterial strain (*Escherichia coli, Bacillus subtilis,* and the like), a yeast strain (*Saccharomyces cerevisiae, Pichia pastoris, Kluyveromyces lactis, Hansenula polymorpha,* and the like), a transgenic plant (dicotyledons or monocotyledons), an insect cell, for example, baculovirus, a mammal cell (COS cells, CHO cells, C127 cells, HeLa cells, and the like) and a non-human transgenic animal (for example, a mouse, a cow, a goat, a rabbit, a pig, etc.).

In another preferred embodiment of this aspect of the invention, the host of step (b) is expression bacteria. More preferably, the expression bacteria are BL21(DE3). Expression bacteria BL21(DE2) are chemically competent *Escherichia coli* cells having a genotype suitable for protein expression and transformation and a known genome (Genome sequences of *Escherichia coli* B strains REL606 and BL21(DE3). Jeong H, et al. J. Mol. Biol. 2009 Dec 11).

A competent bacterium is characterized by having a weakened bacterial wall, and is therefore more likely to capture foreign DNA by means of a heat or electric shock (transformation) process. Expression bacteria are used for protein production. In this specification, expression bacteria are those having the machinery required for over-expressing the inserted cDNA and producing the recombinant protein.

In another preferred embodiment of this aspect of the invention, the nucleotide sequence SEQ ID NO: 1 of step (a) is integrated by means of a ligation process.

To perform the ligation process, the insert:plasmid mixture was resuspended in the product called In-Fusion Dry-Down pellet (Clontech). In-Fusion Dry-Down pellet is a lyophilisate containing the In-Fusion enzyme, which favors the binding of the insert to the plasmid as a result of the nucleotide sequence homology present in both of them.

Therefore, in another preferred embodiment of the invention a lyophilisate comprising the In-Fusion enzyme is used in ligation. This enzyme is a poxvirus DNA polymerase with 3'-5' exonuclease activity, which is capable of binding single-strand DNA molecules having short homologous sequences at their ends, such as an amplified PCR product and a vector.

In another preferred embodiment, the insert was synthesized using primers with the nucleotide sequence SEQ ID NO: 3 and SEQ ID NO: 4.

Another aspect relates to:
- the soluble IFNAR2 protein, sIFNAR2, or IFNAR2.3,
- the amino acid sequence SEQ ID NO: 2 or an amino acid sequence with an identity of at least 80%, 90%, 95%, 98%, or 99% with SEQ ID NO: 2 and in which the peptide encoded by said amino acid sequence exhibits the activity and the structural characteristics of the sIFNAR2 protein,
- the recombinant protein of the invention obtained by means of the method of the invention, or
- the sIFNAR2 protein obtained by other non-recombinant means,
for use thereof in the prevention, control, treatment, and/or alleviation of a viral disease; or alternatively it relates to the use of a protein comprising the amino acid sequence SEQ ID NO: 2 or to the recombinant protein of the invention, or the protein of the invention (soluble IFNAR2, sIFNAR2, or IFNAR2.3) obtained by other non-recombinant means, in the preparation of a medicinal product for preventing, controlling, treating, and/or alleviating a viral disease. The protein of the invention (soluble IFNAR2, sIFNAR2, or IFNAR2.3) is preferably a recombinant protein, and even more preferably obtained by means of the method described in the present invention because even though it can be obtained by means of any method known in the state of the art for obtaining proteins, the methods for obtaining and purifying protein that have been described herein are advantageous.

Non-recombinant means for obtaining peptides or proteins are known to one having average skill in the art, and can be, for example, without limitation, by means of chemical synthesis either in solution or in solid phase.

The term "viral disease" refers to the clinical manifestation of a viral infection. A viral infection refers to the contamination, immunological response, and structural damage of a host caused by a virus, i.e., invasion occurs with cell or tissue injury caused by said virus or its products. The infection can be local or systemic.

The term "virus" refers to a microscopic, acellular infective agent which is only capable of multiplying inside the cells of other organisms.

Viruses are classified in different groups:
- Group I: double-stranded DNA virus.
- Group II: single-stranded DNA virus.
- Group III: double-stranded RNA virus.
- Group IV: positive single-stranded RNA virus.
- Group V: negative single-stranded RNA virus.
- Group VI: single-stranded reverse-transcribing RNA virus.
- Group VII: double-stranded reverse-transcribing DNA virus.

Viral diseases that can be prevented, controlled, treated, and/or alleviated with the recombinant protein of the invention are preferably selected, without limitation, from the list comprising: hepatitis A virus (HAV), hepatitis B virus (HBV), hepatitis C virus (HCV), hepatitis D virus (HDV), hepatitis E virus (HEV), hepatitis F virus, or hepatitis G virus (HGV), human immunodeficiency virus (HIV), respiratory syncytial virus, influenza virus, encephalomyocarditis virus, vesicular stomatitis virus, or any combinations thereof.

HAV is a group IV virus belonging to the Picomaviridae family and the genus *Hepatovirus.* HBV is a group VII virus belonging to the Hepadnaviridae family and the genus *Orthohepadnavirus.* HCV is a group IV virus belonging to the Flaviviridae family and the genus *Hepacivirus.* HDV is a group V virus belonging to the Deltaviridae family and the genus *Deltavirus.* HEV is a group IV virus belonging to the Hepeviridae family. HFV is a single-stranded DNA virus. HGV is an RNA virus also known as "GB virus C" or "GBVC".

HIV is a group VI virus belonging to the Retroviridae family and the genus *Lentivirus.* There are two species of this virus: HIV Type 1 and HIV Type 2.

Respiratory syncytial virus (VSR) is a negative-sense single-stranded RNA virus of the Paramixovirus (Paramyxoviridae) family, which includes common respiratory viruses such as those causing measles and mumps. VSR is a member of the Pneumovirus subfamily.

Influenza virus is a group V virus of the Orthomyxoviridae family. There are different genera of viruses which cause influenza: *Influenzavirus A, Influenzavirus B,* and *Influenzavirus C.*

Encephalomyocarditis virus is an RNA virus belonging to the genus *Cardiovirus* of the family. *Enterovirus, rinovirus, aftovirus,* and *hepatovirus* belong to the same family.

Vesicular stomatitis virus is a group V virus belonging to the Rhabdoviridae family and the genus *Vesiculovirus.* There are two serotypes of vesicular stomatitis virus which are split into different subtypes.

### USES OF THE ANTIBODIES AND COMPOSITIONS OF THE INVENTION

As demonstrated in the examples of the invention, the recombinant protein of the invention and/or the sIFNAR2 protein can be used for preventing, controlling, treating, and/or alleviating a viral disease. Furthermore, another object of the present invention relates to the use of antibodies or fragments thereof which are capable of binding to the recombinant protein of the invention. These antibodies or fragments thereof can be readily obtained from antisera.

The antisera for the recombinant protein described in the present invention can be generated by means of standard techniques, for example, by means of injecting the recombinant protein of the invention into a suitable animal and collecting and purifying the antisera of the animals. The antibodies or fragments thereof which bind to SEQ ID NO: 2, or a sequence variant thereof according to the invention, can be identified by means of standard immunoassays. The antibodies thus obtained (hereinafter, antibodies of the invention) can be used for the diagnostic method of the invention. The antibodies or fragments thereof are preferably monoclonal antibodies.

Therefore, in another aspect the invention relates to the use of an antibody or a fragment thereof which specifically recognizes the recombinant protein of the invention, hereinafter antibody of the invention, for use as a medicinal product for preventing, controlling, treating, and/or alleviating a viral disease; or alternatively it relates to the use of an antibody or a fragment thereof which specifically recognizes the recombinant protein of the invention in the preparation of a medicinal product for preventing, controlling, treating, and/or alleviating a viral disease. The antibodies contemplated in the context of the present invention include polyclonal antisera, purified IgG molecules, supernatants, or ascitic fluid containing monoclonal antibodies, Fv, Fab, Fab', and F(ab')₂ fragments, ScFv diabodies, triabodies, tetrabodies, and humanized antibodies.

Another aspect of the invention relates to the use of a composition, hereinafter composition of the invention, comprising:
a) a protein comprising the amino acid sequence SEQ ID NO: 2,
b) the recombinant protein of the invention,
c) the protein of the invention (soluble IFNAR2, sIFNAR2, or IFNAR2.3) obtained by other non-recombinant means, and/or
d) the antibody, or a fragment thereof, of the invention,
in the preparation of a medicinal product for preventing, controlling, treating, and/or alleviating a viral disease.

Said composition can be a pharmaceutical composition. Therefore, another aspect of the invention relates to pharmaceutical compositions, hereinafter pharmaceutical compositions of the invention, comprising at least one of the polynucleotides of the invention, polypeptides of the invention, or the mature form thereof, an antibody of the invention, or a fragment thereof, the recombinant protein of the invention, the protein of the invention (soluble IFNAR2, sIFNAR2, or IFNAR2.3) obtained by other non-recombinant means, and/or along with a pharmaceutically acceptable excipient. For use in medicine, the compounds and combinations of compounds of the invention can be formulated together with an excipient which is acceptable from the pharmaceutical viewpoint. Preferred excipients for use in the present invention include sugars, starches, celluloses, gums, proteins, and others. In a particular embodiment, the pharmaceutical composition of the invention will be formulated, without limitation, in a solid pharmaceutical dosage form (for example, tablets, capsules, coated tablets, pellets, suppositories, etc.) or liquid pharmaceutical dosage form (for example, solutions, suspensions, emulsions, etc.). In another particular embodiment, the pharmaceutical compositions of the invention can be administered by any route, including, without limitation, oral, intravenous, intramuscular, intraarterial, intramedullary, intrathecal, intraventricular, transdermal, subcutaneous, intraperitoneal, intranasal, enteric, topical, sublingual, or rectal route.

"Adjuvants" and "pharmaceutically acceptable vehicles" refer to those substances or combination of substances known in the pharmaceutical sector, used in the preparation of pharmaceutical dosage forms, and include, without limitation, solids, liquids, solvents, or surfactants. Pharmaceutically acceptable vehicles which can be used in the present invention are vehicles known in the state of the art.

The dosage for obtaining a therapeutically effective amount depends on a number of factors, such as, for example, the age, weight, sex, or tolerance of the individual to whom it is administered. In the sense used in this description, the expression "therapeutically effective amount" refers to the amount of the pharmaceutical composition of the invention which produces the desired effect, and it will generally be determined, among other things, by the actual characteristics of said compounds and said pharmaceutical composition and the therapeutic effect to be achieved.

In a preferred embodiment of this aspect of the invention, the composition of the invention comprises another active ingredient. The active ingredient is preferably selected from the list consisting of other antivirals, analgesics, antipyretics, decongestants, or other active ingredients used in the treatment of viral diseases.

In another preferred embodiment, the composition of the invention further comprises a pharmaceutically acceptable vehicle. In another preferred embodiment, the composition of the invention further comprises another active ingredient or therapeutic agent. Said therapeutic agent is preferably selected from an analgesic agent (in the treatment of inflammation and pain) or an anti-infective agent (in the prevention of infection).

In particular, non-limiting examples of therapeutic agents that are useful according to the invention include the following therapeutic categories: analgesics, such as nonsteroidal anti-inflammatory medicinal products, opiate agonists, and salicylates; anti-infective agents, such as anthelmintics, anti-anaerobics, antibiotics, aminoglycoside antibiotics, antifungal antibiotics, cephalosporins, macrolide antibiotics, various beta-lactam antibiotics, penicillins, quinolone antibiotics, sulfonamide antibiotics, tetracycline antibiotics, antimycobacterials, antituberculosis antimycobacterials, antiprotozoals, antimalarial antiprotozoals, antiviral agents, anti-retroviral agents, scabicides, anti-inflammatory agents, anti-inflammatory corticosteroids, local topical anesthetics/antipruritics, anti-infective agents, topical anti-infective antimycotics, topical anti-infective antivirals, electrolytic and renal agents, such as acidifying agents, alkalinizing agents, diuretics, carbonic anhydrase inhibitors, diuretics, loop diuretics, osmotic diuretics, potassium-sparing diuretics, thiazide diuretics, electrolyte replacements, and uricosuric agents, enzymes, such as pancreatic enzymes and thrombolytic enzymes, gastrointestinal agents, such as antidiarrheals, antiemetics, gastrointestinal anti-inflammatory agents, the salicylate of anti-inflammatory agents, anti-ulcer antacids, gastric acid-pump inhibitor anti-ulcer agents, gastric mucosal anti-ulcer agents, anti-ulcer H2 blockers, cholelitholytic agents, digestants, emetics, laxatives and stool softeners, and prokinetic agents, general anesthetics such as halogenated inhalational anesthetics, inhalational anesthetics, intravenous anesthetics, barbiturate anesthetics, intravenous benzodiazepine anesthetics, intravenous opiate anesthetics, and intravenous agonist anesthetics, hormones, and hormone modifiers, such as abortifacients, corticosteroid adrenal agents, adrenal agents, androgens, anti-androgens, immunobiological agents, such as immunoglobulins, immunosuppressants, toxoids, and vaccines; local anesthetics, such as amide local anesthetics and ester-type local anesthetics, musculoskeletal agents, such as anti-gout anti-inflammatory agents, corticosteroid anti-inflammatory agents, gold compound immunosuppressant anti-inflammatory agents, anti-inflammatory agents, nonsteroidal anti-inflammatory drugs (NSAIDs), salicylate anti-inflammatory agents, minerals, and vitamins, such as vitamin A, vitamin B, vitamin C, vitamin D, vitamin E, and vitamin K.

In a particular embodiment, the therapeutic agents that are useful according to the preceding categories include: (1) analgesics in general, such as lidocaine or derivatives thereof and nonsteroidal anti-inflammatory drug (NSAID) analgesics, including diclofenac, ibuprofen, ketoprofen, naproxen; (2) opiate agonist analgesics, such as codeine, fentanyl, hydromorphone, and morphine; (3) salicylate analgesics, such as aspirin (ASA); (4) H1-blocker antihistamines, such as terfenadine, clemastine; (5) anti-infective agents, such as mupirocin; (6) antianaerobic anti-infectives, such as chloramphenicol and clindamycin; (7) antifungal antibiotic anti-infectives, such as amphotericin b, clotrimazole, fluconazole, and ketoconazole; (8) macrolide antibiotic anti-infectives, such as azithromycin and erythromycin; (9) various beta-lactam anti-infectives, such as aztreonam and imipenem; (10) penicillin antibiotic anti-infectives, such as nafcillin, oxacillin, penicillin G, penicillin V; (11) quinolone antibiotic anti-infectives, such as ciprofloxacin and norfloxacin; (12) tetracycline antibiotic anti-infectives, such as doxycycline, minocycline, and tetracycline; (13) antituberculosis antimycobacterial anti-infectives such as isoniazid (INH), rifampin; (14) antiprotozoal anti-infectives, such as atovaquone and dapsone; (15) antimalarial antiprotozoal anti-infectives, such as chloroquine and pyrimethamine; (16) anti-retroviral anti-infectives, such as ritonavir and zidovudine; (17) antiviral anti-infective agents, such as acyclovir, gancyclevir, interferon alpha, and rimantadine; (18) topical antifungal anti-infectives, such as amphotericin B, clotrimazole, miconazole, nystatin; (19) topical anti-infective antivirals, such as acyclevir; (20) electrolytic and renal agents, such as lactulose; (21) loop diuretics, such as furosemide; (22) potassium-sparing diuretics, such as triamterene; (23) thiazide diuretics, such as hydrochlorothiazide (HCTZ); (24) uricosuric agents, such as probenecid; (25) enzymes, such as RNase and DNase; (26) antiemetics, such as prochlorperazine; (27) salicylate gastrointestinal anti-inflammatory agents, such as sulfasalazine; (28) gastric acid-pump inhibitor anti-ulcer agents, such as omeprazole; (29) H2-blocker anti-ulcer agents, such as cimetidine, famotidine, nizatidine, and ranitidine; (30) digestants, such as pancrelipase; (31) prokinetic agents, such as erythromycin; (32) ester local anesthetics, such as benzocaine and procaine; (33) musculoskeletal corticosteroid anti-inflammatory agents, such as beclomethasone, betamethasone, cortisone, dexamethasone, hydrocortisone, and prednisone; (34) musculoskeletal anti-inflammatory immunosuppressants, such as azathioprine, cyclophosphamide, and methotrexate; (35) musculoskeletal nonsteroidal anti-inflammatory drugs (NSAIDs), such as diclofenac, ibuprofen, ketoprofen, ketorlac, and naproxen; (36) minerals, such as iron, calcium, and magnesium; (37) vitamin B compounds, such as cyanocobalamin (vitamin B12) and niacin (vitamin B3); (38) vitamin C compounds, such as ascorbic acid, and (39) vitamin D compounds, such as calcitriol.

As it is used herein, the term "active ingredient," "active substance," "pharmaceutically active substance," or "pharmaceutically active ingredient" means any component that potentially provides pharmacological activity or another different effect in the diagnosis, cure, mitigation, treatment, or prevention of a disease, or that affects the structure or function of the body of humans or other animals. The term includes those components that promote a chemical change in the preparation of the drug and are present therein in a modified form envisaged for providing the specific activity or effect.

In another preferred embodiment of this aspect of the invention, the viral disease is caused by a virus selected from the list consisting of: hepatitis A virus (HAV), hepatitis B virus (HBV), hepatitis C virus (HCV), hepatitis D virus (HDV), hepatitis E virus (HEV), hepatitis F virus, or hepatitis G virus (HGV), human immunodeficiency virus (HIV), respiratory syncytial virus, influenza virus, encephalomyocarditis virus, vesicular stomatitis virus, or any combinations thereof.

As it is used in this specification, the term "medicinal product" refers to any substance used for the prevention, diagnosis, alleviation, treatment, or curing of undesired physiological states in humans and animals.

As it is used herein, the term "individual" refers to animals, preferably mammals, and more preferably humans. The term "individual" does not seek to be limiting in any aspect, where the individual can be of any age, sex, and physical condition.

*Type I (alpha, beta, and omega) interferons carry out their action through interaction with the membrane receptor IFNAR that is formed by two subunits, IFNAR1 and IFNAR2.* The IFNAR2 subunit of the receptor undergoes an alternative mRNA processing which gives rise to three different forms: a short form (IFNAR2b), a functionally active long form (IFNAR2c), and the soluble form (sIFNAR2, IFNAR2.3, or IFNAR2a). only IFNAR2c acts as a functional receptor together with IFNAR1 and is capable of mediating the biological effects of IFNβ through the activation of JAK-STAT cascade signaling.

Multiple transcription variants encoding at least two different isoforms have been found for this gene. The amino acid sequence of sIFNAR2 is found in the GenBank (NCBI) with accession number L41943.1 and in SEQ ID NO: 2. Said SEQ ID NO: 2 is represented by the following amino acid sequence:

MLLSQNAFIFRSLNLVLMVYISLVFGISYDSPDYTDESCTFKISLRNFRSILSWELKNHSIVPTHYT
LLYTIMSKPEDLKVVKNCANTTRSFCDLTDEWRSTHEAYVTVLEGFSGNTTLFSCSHNFWLAID
MSFEPPEFEIVGFTNHINVMVKFPSIVEEELQFDLSLVIEEQSEGIVKKHKPEIKGNMSGNFTYIID
KLIPNTNYCVSVYLEHSDEQAVIKSPLKCTLLPPGQESEFS.

In the context of the present invention, sIFNAR2 is also defined by a nucleotide or polynucleotide sequence which constitutes the coding sequence of the protein that is listed in SEQ ID NO: 2, and would comprise different variants originating from:
a) nucleic acid molecules encoding a polypeptide comprising the amino acid sequence of SEQ ID NO: 2,
b) nucleic acid molecules the complementary strand of which hybridizes with the polynucleotide sequence of a),
c) nucleic acid molecules the sequence of which differs from a) and/or b) due to genetic code degeneration,
d) nucleic acid molecules encoding a polypeptide comprising the amino acid sequence with an identity of at least 60%, 70%, 80%, 90%, 95%, 98%, or 99% with SEQ ID NO: 2, and in which the polypeptide encoded by said nucleic acids exhibits the activity and the structural characteristics of the IFNAR2.3 protein. Said nucleic acid molecules include, among others, the one listed in the GenBank (NCBI) sequence L41943.1 and SEQ ID NO: 1. Said SEQ ID NO: 1 is represented by the following nucleotide sequence:

agatgtaaaagtcaagagaagactctaaaaatagcaaagatgcttttgagccagaatgccttcatcttcagatcacttaatttggttctca
tggtgtatatcagcctcgtgtttggtatttcatatgattcgcctgattacacagatgaatcttgcactttcaagatatcattgcgaaatttccggt
ccatcttatcatgggaattaaaaaccactccattgtaccaactcactatacattgctgtatacaatcatgagtaaaccagaagatttga
aggtggttaagaactgtgcaaataccacaagatcattttgtgacctcacagatgagtggagaagcacacacgaggcctatgtcaccg
tcctagaaggattcagcgggaacacaacgttgttcagttgctcacacaatttctggctggccatagacatgtcttttgaaccaccagagtt
tgagattgttggttttaccaaccacattaatgtgatggtgaaatttccatctattgttgaggaagaattacagtttgatttatctctcgtcattga

agaacagtcagagggaattgttaagaagcataaacccgaaataaaaggaaacatgagtggaaatttcacctatatcattgacaagtt
aattccaaacacgaactactgtgtatctgtttatttagagcacagtgatgagcaagcagtaataaagtctcccttaaaatgcaccctcctt
ccacctggccaggaatcagaattttcataactttttagcctggccatttcctaacctgccaccgttggaagccatggatatggtggaggtc
atttacatcaacagaaagaagaaagtgtgggattataattatgatgatgaaagtgatagcgatactgaggcagcgcccaggacaagt
ggcggtggctataccatgcatggactgactgtcaggcctctgggtcaggcctctgccacctctacagaatcccagttgatagacccgg
agtccgaggaggagcctgacctgcctgaggttgatgtggagctccccacgatgccaaaggacagccctcagcagttggaactcttg
agtgggccctgtgagaggagaaagagtccactccaggacccttttcccgaagaggactacagctccacggaggggtctgggggca
gaattaccttcaatgtggacttaaactctgtgtttttgagagttcttgatgacgaggacagtgacgacttagaagcccctctgatgctatcgt
ctcatctggaagagatggttgacccagaggatcctgataatgtgcaatcaaaccatttgctggccagcggggaagggacacagcca
acctttcccagcccctcttcagagggcctgtggtccgaagatgctccatctgatcaaagtgacacttctgagtcagatgttgaccttgggg
atggttatataatgagatgactccaaaactattgaatgaacttggacagacaagcacctacagggttctttgtctctgcatcctaacttgct
gccttatcgtctgcaagtgttctccaagggaaggaggaggaaactgtggtgttcctttcttccaggtgacatcacctatgcacattcccag
tatggggaccatagtatcattcagtgcattgtttacatattcaaagtggtgcactttgaaggaagcacatgtgcacctttcctttacactaat
gcacttaggatgtttctgcatcatgtctaccagggagcagggttccccacagtttcagaggtggtccaggaccctatgatatttctcttcttt
cgttcttttttttttttttgagacagagtctcgttctgtcgcccaagctggagcgcaatggtgtgatcttggctcactgcaacatccgcctccc
gggttcaggtgattctcctgcctcagcctccctcgcaagtagctgggattacaggcgcctgccaccatgcctagcaaatttttgtatttttag
tggagacaggattttaccatgttggccaggctggtctcgaactcctgacctcaagtgatctgccctcctcagcctcgtaaagtgctggga
ttacaggggtgagccgctgtgcctggctggccctgtgatatttctgtgaaataaattgggccagggtgggagcagggaaagaaaagg
aaaatagtagcaagagctgcaaagcaggcaggaagggaggaggagagccaggtgagcagtggagagaagggggccctgc
acaaggaaacagggaagagccatcgaagtttcagtcggtgagccttgggcacctcacccatgtcacatcctgtctcctgcaattgga
attccaccttgtccagccctccccagttaaagtggggaagacagactttaggatcacgtgtgtgactaatacagaaaggaaacatggc
gtcggggagagggataaacctgaatgccatattttaagttaaaaaaaaaaaa.

The terms "polynucleotide" and "nucleic acid" are used interchangeably herein in reference to polymeric forms of nucleotides of any length, both ribonucleotides (RNAs) and deoxyribonucleotides (DNAs).

The terms "amino acid sequence," "peptide," "oligopeptide," "polypeptide" and "protein" are used interchangeably herein and refer to a polymeric form of amino acids of any length, which can be coding or non-coding, chemically or biochemically modified, amino acids.

Throughout the description and claims, the word "comprises" and variants thereof do not seek to exclude other technical features, additives, components, or steps. For those skilled in the art, other objects, advantages, and features of the invention will be inferred in part from the description and in part from putting the invention into practice. The following examples and drawings are provided by way of illustration and do not seek to be limiting of the present invention

### EXAMPLES OF THE INVENTION

### Example 1: Evaluating the antiviral capacity of recombinant sIFNAR2 by means of the cytopathic effect test or bioassay

This is a technique recommended by the World Health Organization (WHO) for evaluating antiviral capacity and detecting anti-IFNβ antibodies. This technique is based on the antiviral capacity of IFNβ, such that a cell culture (line A549), when infected with a specific virus (encephalomyocarditis virus), is protected by the presence of IFNβ, unless neutralizing antibodies blocking the action of said IFNβ are present in the serum of the patient.

A series of bioassays were performed in the presence of recombinant sIFNAR2 that have allowed confirming through said bioassays that the recombinant sIFNAR2 of the invention has a significant antiviral capacity, where the cells remain alive and the recombinant sIFNAR2 acts in a manner similar to IFNβ.

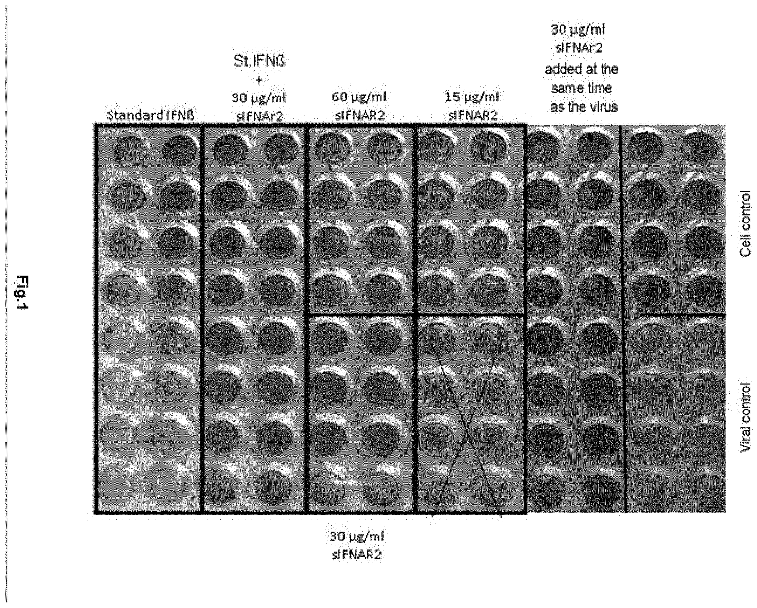

Figure 1 illustrates a representative bioassay conducted in the presence of sIFNAR2. The more intense violet color corresponds with greater cell survival after infecting the cell culture with the encephalomyocarditis virus (EMC). The different experimentation scenarios contemplated in Figure 1 are as follows:
- Columns 1, 2: Standard IFNβ. The test starts with an IFNβ concentration of 20 IU and serial dilutions are gradually performed. As the concentration gradually decreases, color degradation is observed because the cells are less protected by the action of IFNβ.
- Columns 3, 4: IFNβ, diluting from 20 IU, and a constant concentration of 30 ug/ml of sIFNAR2 are added along the entire column. The cells remain alive in all the dilutions, no color degradation being observed in any well. As the IFNβ gradually stops to perform its protective function due to the dilution thereof, sIFNAR2 continues to protect the cells from the virus.
- Columns 5-6, top part: 8 replicates with 60 ug/ml of sIFNAR2 added. It can be seen that the cells are still alive, being protected with respect to the VC.
- Columns 5-6, bottom part: 8 replicates with 30 ug/ml of sIFNAR2 added. It can be seen that the cells are still alive, being protected with respect to the viral control.
- Columns 7-8, top part: 8 replicates with 15 ug/ml sIFNAR2 added. It can be seen that the cells are still alive, being protected with respect to the VC.
- Columns 9-10: 30 ug/ml of sIFNAR2 have been added in the entire column on the third day of the assay, and the virus is added at the same time, a higher antiviral capacity therefore being observed.
- Columns 11-12, top part: Cell control (CC): only cells A549;
- Columns 11-12, bottom part: Viral control (VC): cells A549 infected with encephalomyocarditis virus, without protection.

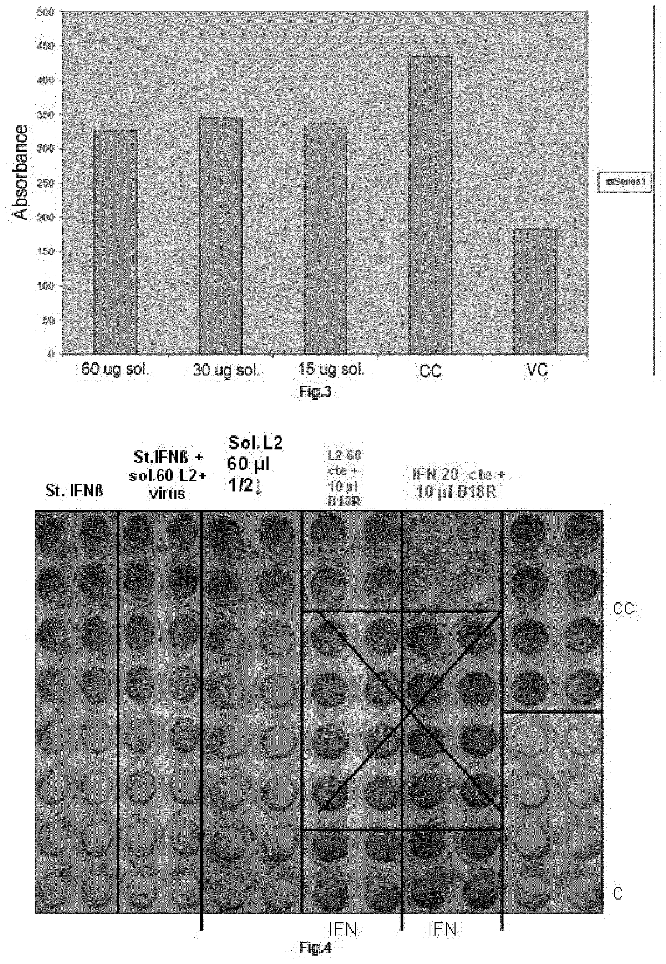

Figure 4 consists of a bioassay dish showing the antiviral capacity of sIFNAR2. It is demonstrated that B18R, an IFNβ inhibitor, blocks the action of IFNβ but not the action of the sIFNAR2 recombinant protein; the manner in which the cells remain protected in the presence of B18R+sIFNAR2 can be seen.
- Columns 1, 2: Standard IFNβ. The test starts with an IFNβ concentration of 20 IU and serial dilutions are gradually performed. As the concentration gradually decreases, color degradation is observed because the cells are less protected by the action of IFNβ.
- Columns 3, 4: Combination of IFNβ /sIFNAR2. IFNβ, diluting from 20 IU, and sIFNAR2, diluting from a concentration of 60 ug/ml, are added. The manner in which the presence of the two molecules slightly increases protection with respect to the presence of each of them separately can be seen.
- Columns 5-6: 60 ug/ml of sIFNAR2 is added in the first well and serial dilutions are gradually performed. It can be seen that the cells are still alive in the first wells and that viability gradually decreases as the concentration of sIFNAR2 gradually decreases.
- Columns 7-8, top part: 4 replicates with 60 ug/ml of sIFNAR2 and B18R (a potent IFNβ inhibitor) added. It can be seen that the cells are still alive, being protected with respect to the VC. The IFNβ inhibitor does not inhibit sIFNAR2.
- Columns 9-10, top part: 4 replicates with IFNβ 20 U and B18R (a potent IFNβ inhibitor) added. It can be seen that the cells are dead given that B18R inhibits IFNβ.
- Columns 7-8, bottom part: 4 replicates with IFN80 (added on the same day as the virus). Protection is observed.
- Columns 9-10, top part: 4 replicates with IFN40 (added on the same day as the virus). Protection is observed.
- Columns 11-12, top part: Cell control (CC): only cells A549.
- Columns 11-12, bottom part: Viral control (VC): cells A549 infected with the encephalomyocarditis virus, without protection.

### Example 2: Producing the sIFNAR2 recombinant protein

### Selecting the cloning vector

The chosen prokaryotic expression system is the prelinearized vector pEcoli-Cterm 6xHN Linear (Clontech). The resulting protein will have a histidine-asparagine tag fused thereto at the carboxy terminal end which will be useful for its purification.

The drawings show in detail the structure of the vector into which the insert with the nucleotide sequence of the protein of interest of the present invention was integrated.

The pEcoli Cterm 6xHN Linear expression system is based on the expression system of the strong T7 promoter, controlled by the LacZ operon which is in turn inducible by IPTG (isopropyl-β-D-thiogalactopyranoside). Furthermore, the plasmid has an ampicillin-resistance gene which allows selecting clones containing the plasmid.

The machinery of the expression bacteria BL21 (DE3) using the T7 promoter was used for protein production. Bacteria BL21 (DE3) contain a chromosomal copy of the T7 RNA polymerase gene which is in turn under the control of the IPTG-inducible lacUV5 promoter.

### Synthesizing the DNA insert

The first stage of designing the cloning strategy was insert synthesis. To that end, all the information about the SIFNAR2 sequence, such as the signal sequence of the peptide, the post-translational modifications, the biochemical characteristics of the protein, the domains thereof, etc., was collected. All this information was obtained from the UNIPROT database (http://www.uniprot.org/uniprot/P48551) hosting amino acid sequences of proteins and their biochemical characteristics.

The SIFNAR2 mRNA sequence was obtained from the NCBI NUCLEOTIDE database (http://www.ncbi.nlm.nih.gov/nuccore.)
Following the manufacturer's guidelines, the primers must comply with the following requirements:
The 5' end must:
   - contain 15 bases that are homologous to the 15 bases at the end of the DNA fragment of the vector into which it will be inserted.
The 3' end must:
   - have 15 bases that are homologous to the ends of the gene that will be inserted;
   - have a length between 18-25 bp and a GC content of 40-60%;
   - have no start codon (ATG) and stop codon in the sequence to be amplified;
   - have no signal sequence.

Taking into account these premises, the primers yielded a 638-bp product after amplification. The sequences of the primers were as follows:
Sense (sequence SEQ ID NO. 3):
   5'TAAGGCCTCTGTCGACATTTCATATGATTCGCCTGATTACACGATG3'
Antisense (sequence SEQ ID NO. 4):
   5'CAGAATTCGCAAGCTTTGAAAATTCTGATTCCTGGCCAGGTGGAA 3'

The insert was synthesized by means of conventional PCR from the primers designed in the preceding stage using a high fidelity Taq and using a cDNA originating from a commercial human cDNA mixture as a template. The optimum concentration, temperature, and time conditions for synthesizing the insert were as follows:

**Table 1. Summary of conventional PCR reagents for insert synthesis**

| **Reagents** | **Volume/sample** | **Final concentration/sample** |
|---|---|---|
| **Rnase-free water** | 40 µl | |
| **Sense primer (20 µM)** | 1 µl | 0.4 µM |
| **Antisense primer (20 µM)** | 1 µl | 0.4 µM |
| **Dntp (10 µM)** | 1 µl | 0.2 µM |
| **Buffer 5X** | 5 µl | 1X |
| **Pfu High Fidelity** | 1 µl | |
| **Cdna** | 1 µl | |

### Temperature conditions:

**Table 2. Summary of temperature conditions for insert synthesis by means of conventional PCR**

| **Stage** | **Temperature** | **Time** | **Cycles** |
|---|---|---|---|
| **Initial denaturation** | 95°C | 3 minutes | 1 |
| **Denaturation** | 95°C | 20 seconds | 40 cycles |
| **Annealing** | 60.4°C | 20 seconds | |
| **Extension** | 72°C | 30 seconds | |
| **Final extension** | 72°C | 10 minutes | 1 |
| **Final stage** | 4°C | Infinite | |

The end product obtained from the PCR was separated according to size by means of the horizontal electrophoresis technique in 2% agarose gel dissolved in TAE buffer, together with Gold View Nucleic Acid Stain intercalator (Sbs Genetech) at a 1/20 dilution. The gel was subjected to a constant current of 80 V and viewed under a UV transilluminator which allowed locating the band of interest according to the number of base pairs. The band located at the height of 638 bp was taken out from the agarose gel with the help of a scalpel.

The amplified insert sequence contained in the agarose was purified with the QIAquick Gel Extraction commercial kit (QIAGEN) following the manufacturer's indications. At the end of the process, an eluate was obtained and it was quantified with a spectrophotometer (Nanodrop, Thermo) before being stored at -20°C.

### Ligation process

Following the work diagram, the next stage of the cloning process was the ligation process, i.e., "gluing" the sIFNAR2 nucleotide sequence to the plasmid, giving rise to the recombinant protein.

To determine the concentrations and volumes of the insert and plasmid, the company, Clontech, offers on its website a computer-based tool (http://bioinfo.clontech.com/infusion) for calculating the amounts of the optimum amounts of vector and insert for the ligation process based on known vector and insert length variables.

To perform the ligation process, the insert:plasmid mixture was resuspended in the product called In-Fusion Dry-Down pellet (Clontech). In-Fusion Dry-Down pellet is a lyophilisate containing the In-Fusion enzyme, which favors the binding of the insert to the plasmid as a result of the nucleotide sequence homology present in both of them. The ligation reaction was carried out in a thermocycler at 37°C for 15 minutes, followed by 15 minutes at 50°C, and then transferred to ice. Finally, the ligation product was resuspended in 40 µl of pH 8 TE buffer (Tris-HCI, EDTA).

### Transforming into replicative bacteria

The competent bacteria used were MAX Efficiency DH5α™ Competent Cells (Invitrogen) which were transformed with the plasmid according to the following protocol:
As a positive control of the transformation technique, 5 µl of plasmid pUC19 (positive control) were added in an aliquot of competent bacteria and this mixture was gently resuspended. The bacteria were simultaneously transformed with the ligation product. To that end, 2.5 µl were added to an aliquot of competent bacteria and gently mixed. Both aliquots of (control and test) bacteria were then incubated for 30 minutes in ice. After this time, the samples were subjected to heat shock at 42°C for 45 seconds. The samples were quickly transferred to ice for 2 minutes and 900 µl of SOC medium (which favors the transformation process) were then added. For the plasmid to express resistance to ampicillin, the samples were incubated at 37°C for 1 hour while stirring at 225 rpm. Finally, the transformed bacteria were seeded at different volumes in LB agar dishes supplemented with 100 µg/ml of ampicillin and incubated overnight at 37°C.

### Purifying the plasmid DNA and verifying the reading frame

After one night in the incubator, the bacteria had formed CFUs (colony forming units). To evaluate the characteristics of each CFU, the CFUs were isolated separately with the help of a seeding wire and seeded in tubes with 4 ml of LB broth medium supplemented with 100 µg/ml of ampicillin. These suspensions were incubated overnight at 37°C while stirring at 220 rpm together with a negative control which was a tube of LB broth without bacteria. The plasmid contained in the bacteria was then purified following the indications of the Promega kit (PureYield™ Plasmid Miniprep System) as explained below:
The bacterial culture was aliquoted into 1.5 ml tubes and centrifuged at 16000 g for 30 seconds in a microcentrifuge. The supernatant of the obtained product was discarded and the precipitate thereof was resuspended in 600 µl of water to which 100 µl of cell lysis buffer were added, and it was mixed by turning upside down. 350 µl of neutralizing solution were added to this mixture and it was again mixed by turning upside down. It was then centrifuged at 16000 g for 3 minutes. The supernatant obtained was transferred to one of the minicolumns provided by the kit which retains the DNA. It was again centrifuged at 16000 g for 15 seconds. 200 µl of washing solution were then added to the minicolumn and it was again centrifuged for 15 seconds. 400 µl of washing solution were then added to the minicolumn and it was centrifuged for 30 seconds. Finally, to elute the DNA which has been retained in the membrane, the minicolumn was transferred to a clean 1.5 ml microcentrifuge tube, 30 µl of sterile water were added to the center of the membrane, and it was incubated for 1 minute at room temperature. Finally, it was centrifuged at 16000 g for 15 seconds to obtain the purified plasmid DNA. The plasmid DNA was quantified by means of absorbance in the spectrophotometer (Nanodrop, Thermo) and stored at -20°C until the time of use.

In this stage, the authors of the present invention obtained different isolated and frozen CFUs, but they did not know whether or not the plasmid contained the insert, its complete sequence, the orientation in the open reading frame, etc., so it was necessary to check that the plasmid complied with all the desired requirements. To that end, two tests were performed:
- Conventional PCR using the plasmid DNA as template DNA.
- DNA sequencing: Plasmids found to be positive in the PCR were sequenced to obtain the nucleotide sequence which would allow evaluating the sequence of the insert and checking the orientation thereof.

The insert sequencing covered upstream sequences coinciding with the T7 promoter and downstream sequences coinciding with the terminal T7 sequence. The obtained sequences were aligned in the 5'→3' direction with the reference sequence with NBCI *GenBank* number CAA61940.1 by means of Multalin bioinformatic software. The results obtained after alignment which verified the integrity of the sequence and the orientation in the correct reading frame are shown below:

### Transforming into expression bacteria BL21 (DE3)

Once the clone containing the plasmid with the correct conditions was verified, the plasmid was transformed into expression bacteria BL21(DE3) for producing the sIFNAR2 recombinant protein, following the same protocol described above for transforming into replicative bacteria and detecting the plasmid.

### Inducing expression of the sIFNAR2 recombinant protein

Under normal conditions, the recombinant protein is not being expressed in plasmid-transformed BL21 (DE3) bacteria because the expression thereof is repressed by the Lac repressor (Lacl) which is bound to the Lac operon. To allow its expression, it is necessary to add IPTG which acts as an inducer, sequestering the repressor and allowing the T7 RNA polymerase to bind to the T7 promoter and perform the transcription process. The following protocol was followed to induce expression of the sIFNAR2 recombinant protein:
A day before inducing protein production, a preculture was prepared in the following manner:
- Bacteria BL21 (DE3) with the plasmid were cultured in 4 ml of LB broth supplemented with ampicillin at a final concentration of 100 µg/ml and incubated overnight at 37°C while stirring at 220 rpm.
   - Protein expression was induced the next day. To that end, the culture of the preceding day was diluted 1/10 in a final volume of 50 ml of LB broth medium supplemented with ampicillin and incubated at 37°C while stirring at 220 rpm until reaching an optical density (OD) of 0.80-1 nm. The IPTG inducer was added at this point at a (pre-established) final concentration of 0.5 mM and the culture was incubated for 4 hours at 37°C while stirring at 220 rpm. The transcription process for protein expression began from this point onwards. After 4 hours of induction (previously optimized), the culture was collected and centrifuged at 1600 g at 4°C for 20 minutes. The supernatant was discarded and the pellet kept at -80°C until subsequent use.

### Extracting the recombinant protein

The expressed recombinant protein was located inside the bacterium. To access said recombinant protein and to be able to purify same, it was necessary to break the bacterial wall by means of physical and chemical processes that are described in detail below:
The bacterial precipitate stored at -80°C was thawed at room temperature. 0.5 ml of bacterial lysis buffer were then added per milliliter of initial culture and it was resuspended with the help of a pipette. The resulting suspension was incubated for 1 hour at room temperature under rotation. After this time has lapsed, the sample was subjected to ultrasounds in cycles of 5 30-second pulses in ice, and with an intensity of 40%. It was then ultra-centrifuged at 15000 g for 20 minutes at 4°C and the membranes of the proteins released from the bacterium were thereby separated. After ultracentrifugation, the supernatant was collected and passed through a 0.45 µm filter.

### Purifying the sIFNAR2 recombinant protein

The product obtained after the extraction contained the recombinant protein together with other bacterial proteins. The affinity chromatography technique was used to purify and isolate the sIFNAR2 recombinant protein, such that the sIFNAR2 recombinant protein is retained as a result of the histidine-asparagine tag it contains. The selected columns have a volume of 1 ml and are filled with sepharose resin which has nickel ions bound thereto. The nickel ions confer upon said resin the capacity to retain histidine-rich proteins, and therefore the sIFNAR2 recombinant protein will be retained, among others. The protein is released from the resin through the addition of a buffer that is rich in imidazol which competes for the nickel-binding site. The protocol that was followed is described in detail below:
Before starting the affinity chromatography purification process, the resin was washed and equilibrated with 10 ml of equilibration buffer. The protein extract containing the protein of interest of the invention was then contacted with the resin under rotation at 4°C for 1 hour, and the resin was then packed into the column. To eliminate proteins that are not bound to the resin, the resin was washed with 10 ml of equilibration buffer. Finally, the proteins retained by nickel were eluted with 5 ml of imidazol-rich elution buffer and collected in aliquots of 1 ml.

### Detecting the recombinant protein: Electrophoresis and Western blot

The first step for detecting the protein was to perform polyacrylamide gel electrophoresis and to then transfer the proteins to a membrane. The protocol that was followed was the following:
The samples were resuspended in 5x loading buffer and boiled at 100°C for 3 minutes in a thermoblock. The samples were then loaded in a 12% polyacrylamide gel submerged in electrophoresis buffer and subjected to a constant current of 130 V. Once electrophoresis ended, the gel obtained was submerged in transfer buffer for several minutes.

The transfer was performed in a semi-dry system in graphite sheets which had been previously wetted with water. The nitrocellulose membrane with pore size of 0.45 µm was then activated by submerging it in water and then equilibrated in transfer buffer. The sandwich was then assembled; 9 sheets of transfer paper previously wetted in transfer buffer were placed on the graphite sheet, the membrane was then placed on top followed by the gel that was going to be transferred. To finish off the sandwich, 9 sheets of transfer paper wetted in transfer buffer were placed thereon again. The transfer was performed for 45 minutes with an intensity of 0.8 mA/cm².

Once the transfer ended, the membrane was separated and blocked with blocking buffer for 2 hours at room temperature while stirring. Blocking is a step which prevents non-specific binding of the antibodies to the free sites of the membrane, these sites being blocked with milk casein. After blocking, the membrane was contacted with the human anti-IFNAR2 primary antibody produced in rabbit (Abnova) with a pre-established dilution of 1/5000 in a blocking solution overnight at 4°C under rotation. The membrane was removed from the solution with antibody the next day and washed with washing buffer. The membrane was incubated for one and a half hours with the rabbit anti-IgG antibody (Sigma-Aldrich) labeled with alkaline phosphatase, at a dilution of 1/10000 in a blocking solution. It was washed in the same way as in the preceding stage. To see the result of the Western blot, the membrane was developed by contacting it with a mixture formed by 200 µl of NBT/BCIP + 10 ml of developing solution at room temperature until a color product appears. Finally, the reaction was stopped by discarding the developing solution and submerging same in a stop solution that is rich in magnesium ions which block the development of the colorimetric reaction by removing NBT/BCIP.

### Analyzing the recombinant protein

The sIFNAR2 recombinant protein was analyzed after purification. To that end, the protein band/bands were cleaved from an SDS/PAGE acrylamide gel and fragmented to perform the subsequent peptide fingerprint analysis by means of MALDI-TOF/TOF mass spectrometry.

### Example 3. A549/encephalomyocarditis virus bioassay with different batches of sIFNAR2 protein

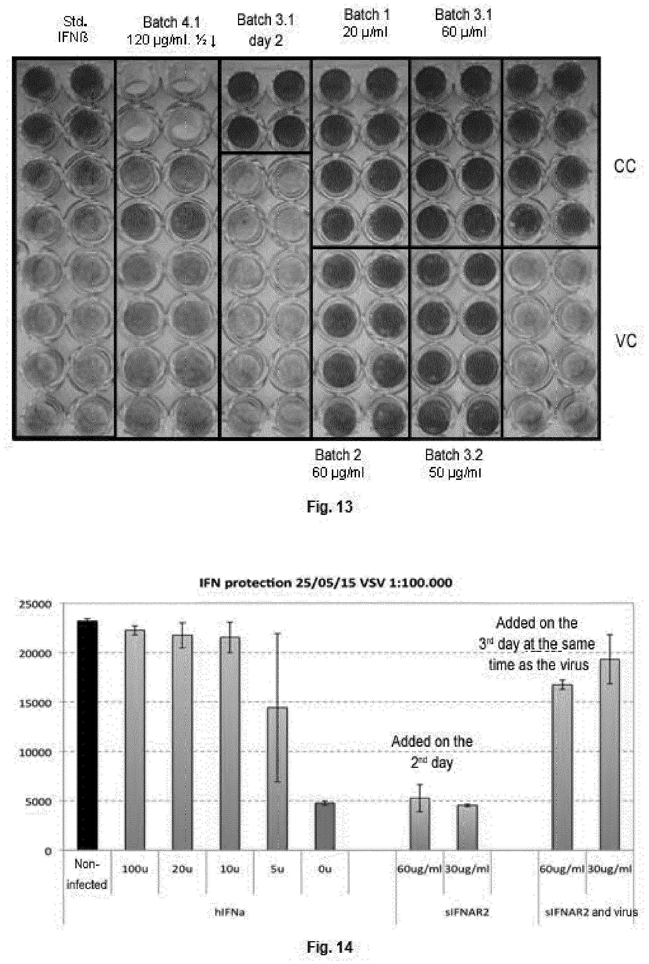

Figure 13 shows the following information:
Columns 1, 2: Standard IFNβ. The test starts with an IFNβ concentration of 20 IU and serial dilutions are gradually performed. As the concentration gradually decreases, color degradation is observed because the cells are less protected by the action of IFNβ.
- Columns 3, 4: Titration of batch 4. As this is the first test, it starts with a concentration of 120 ug/ml, with ½ dilutions being performed. The first concentrations are toxic and do not allow monolayer growth. Culture protection is observed at 30 and 15 ug/ml, with cells stained blue being observed.
- Columns 5-6: 4 replicates with batch 3.1 of 60 ug/ml IFNAR2.3 added on the second day of the assay. It can be seen that the cells are still alive, being protected with respect to the VC.
- Columns 7-8, top part: 8 replicates with batch 1 of 20 ug/ml IFNAR2.3 added. It can be seen that the cells are still alive, being protected with respect to the VC.
- Protection is likewise observed with batches 1, 2, 3.1, and 3.2 added on the third day of the assay (columns 7 to 10). A constant concentration of each of these batches was used because they have been previously titrated.
- Columns 11-12, top part: Cell control (CC): only cells A549;
- Columns 11-12, bottom part: Viral control (VC): cells A549 infected with the encephalomyocarditis virus, without protection.

This bioassay dish demonstrates that sIFNAR2 protection is not an isolated event but rather occurs with the different batches of protein, where it can be clearly observed that the cells are protected from the action of the virus in the presence of sIFNAR2.

### Example 4. HEP2 line/vesicular stomatitis virus (VSV) bioassay

Figure 14 shows the results of a bioassay performed with the HEP2 cell line and vesicular stomatitis virus at Centro Severe Ochoa, CSIC, Madrid. Cell viability is determined with MTT. The dish included a non-infected cell control, a standard IFNβ (100, 20, 10, 5 IU), and a viral control (without IFNβ). Furthermore, two concentrations of recombinant sIFNAR2 added on the second and third day of the assay were included.

Like the observation made for the bioassays performed in Malaga, the presence of sIFNAR2 added on the third day of the assay protects the cell culture from the action of the virus, with the monolayer remaining intact.

### Example 5. Experiments with human immunodeficiency virus (HIV) in the presence of recombinant sIFNAR2

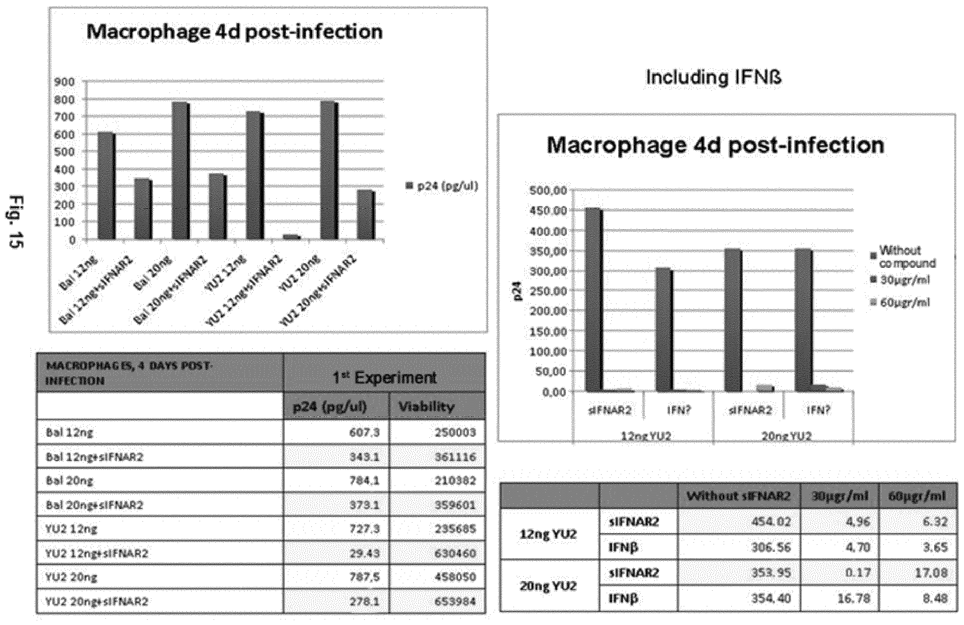

Figure 15 shows the post-infection results of, on one hand, macrophages differentiated from peripheral blood monocytes by adherence under the following conditions:
- with two macrophagotropic HIV viral strains (Bal and YU2)
- at two viral doses of 12 ng and 20 ng of p24 per well
- with one dose of sIFNAR2
- with viral production collected by quantifying the p24 soluble in the supernatant (HIV core) four days after infection.

The overall effect is a decrease in infection of between 30 and 70% with respect to infection without sIFNAR2. Variability exists but the trend is consistent. Viability is not altered, rather it seems to be protected.

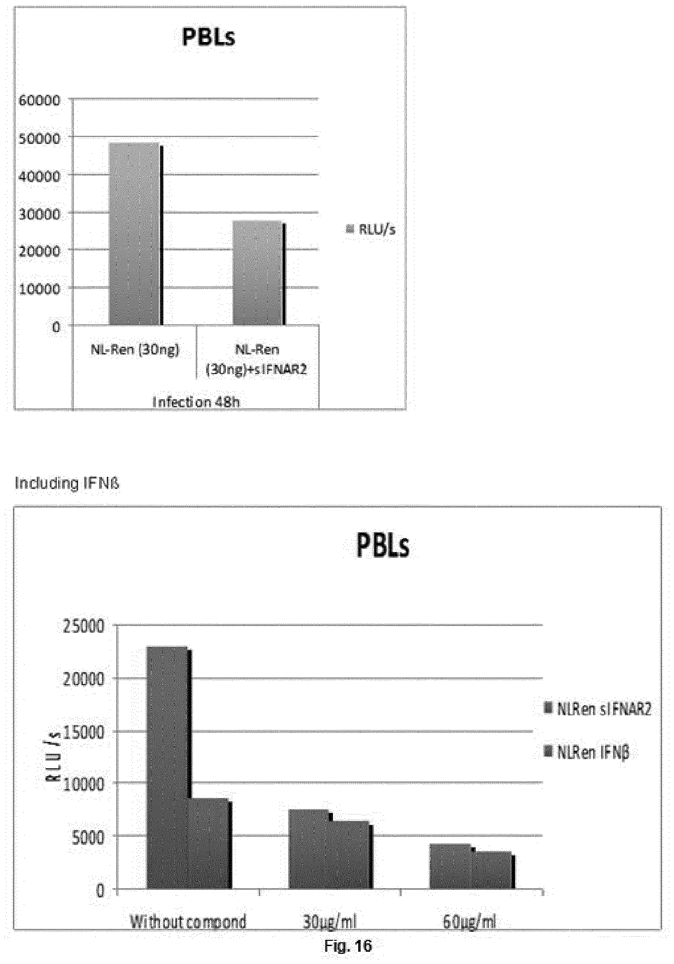

On the other hand, as shown in Figure 16 peripheral blood lymphocytes activated with PHA+IL2 were infected with a luciferase-expressing viral vector. The expression of luciferase under the control of the promoter and regulation of HIV itself is therefore measured. A decrease in infection of about 50% is observed at 48 hours of infection.

### Example 6. Experiments with human respiratory syncytial virus (hRSV) and human metaneumovirus (hMPV) in the presence of recombinant sIFNAR2

As shown in the Figure 17, Vero cells were treated with the indicated amounts of sIFNAR2, IFNβ, or the indicated mABs. 101F is a monoclonal antibody (mAb) which neutralizes human respiratory syncytial virus (hRSV) and human metaneumovirus (hMPV) and 1P is an irrelevant antibody (negative control). After 48 hours, the cells were infected with a fixed amount of GFP-expressing recombinant viruses (hRSV-GFP or hMNV-GFP) in the presence of sIFNAR2, IFNβ, or mABs. The same amount of each of these reagents was again added to the pretreated cultures at the time of infection. Furthermore, an MTT viability assay was performed at 48 hours in parallel in non-infected Vero cell cultures that were, however, treated with sIFNAR2, IFNβ, or mABs,.

Inhibition by sIFNAR2 and mABs is shown in the top panels and inhibition by INF is shown in the bottom panels (the units of the x-axis are different). Viability results are shown in the panels on the right.

IFNAR inhibited, in a very significant manner, the infectivity of hRSV and hMPV at 48 hours, referred to as the percentage of virus control alone. Viability calorimetrically measured at 48 hours in treated, but not infected, cells decreased slightly only at a higher dose of IFNAR.

### Summary

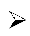 Recombinant sIFNAR2 exhibits, *per se,* antiviral capacity, protecting cells from the action of the virus (VSV or encephalomyocarditis virus) determined by means of a bioassay performed in two independent laboratories. 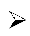 Protection is greater when sIFNAR2 is added on the same day as the infection with the
virus. 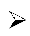 The B18R inhibitor inhibits the action of IFNβ but not the action of sIFNAR2.
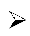 sIFNAR2 has shown antiviral activity against viruses that are relevant to human health such as HIV, RSV, and MNV.

## Claims

1. Use of the sIFNAR2 protein in the preparation of a medicinal product for preventing, improving, treating, and/or alleviating a viral disease.

2. Use of the sIFNAR2 protein according to the preceding claim, wherein the amino acid sequence of the sIFNAR2 protein is SEQ ID NO:2, or an amino acid sequence with an identity of at least 80%, 90%, 95%, 98%, or 99% with SEQ ID NO: 2, and wherein the protein encoded by said amino acid sequence exhibits the activity and the structural characteristics of the sIFNAR2 protein.

3. Use of the sIFNAR2 protein according to any of claims 1 to 2, wherein the sIFNAR2 protein is the recombinant protein of the invention obtained by means of the method described in any of claims 10 to 16.

4. Use of the sIFNAR2 protein according to any of claims 1 to 3, wherein the sIFNAR2 protein is obtained by non-recombinant means.

5. Use of a composition comprising the sIFNAR2 protein as described in any of claims 1 to 4 in the preparation of a medicinal product for preventing, improving, treating, and/or alleviating a viral disease.

6. Use of the composition according to the preceding claim, wherein said composition is a pharmaceutical composition further comprising a pharmaceutically acceptable vehicle and/or pharmaceutically acceptable excipients.

7. Use of the composition according to any of claims 5 or 6, further comprising another active ingredient.

8. Use of the composition according to the preceding claim, wherein the active ingredient is selected from the list consisting of other antivirals, analgesics, antipyretics, decongestants, or other active ingredients used in the treatment of viral diseases.

9. Use of a composition according to any of claims 6 to 8, wherein the viral disease is caused by a virus selected from the list consisting of: hepatitis A virus (HAV), hepatitis B virus (HBV), hepatitis C virus (HCV), hepatitis D virus (HDV), hepatitis E virus (HEV), hepatitis F virus, or hepatitis G virus (HGV), human immunodeficiency virus (HIV), respiratory syncytial virus, influenza virus, encephalomyocarditis virus, vesicular stomatitis virus, or any combinations thereof.

10. A method for obtaining a recombinant protein comprising the following steps:
a) integrating an insert with the nucleotide sequence SEQ ID NO. 1 in an expression vector,
b) transforming a host with the expression vector of step (a),
c) inducing expression of the recombinant protein,
d) extracting the recombinant protein, and optionally
e) purifying the recombinant protein.

11. The method according to the preceding claim, wherein the expression vector is the prelinearized vector pEcoli-Cterm 6xHN Linear.

12. The method according to any of claims 1 to 2, wherein the host of step (b) is expression bacteria.

13. The method according to the preceding claim, wherein the expression bacteria are the bacteria *E.coli* BL21 (DE3).

14. The method according to any of claims 1 to 4, wherein the integration of step (a) is performed by means of a ligation process.

15. The method according to the preceding claim, wherein a lyophilisate comprising the In-Fusion enzyme is used in the ligation.

16. The method according to any of claims 1 to 6, wherein the insert was synthesized using primers with the nucleotide sequence SEQ ID NO: 3 and SEQ ID NO: 4.


SEQUENCE LISTING

<110>  SERVICIO ANDALUZ DE SALUD
       UNIVERSIDAD DE MÁLAGA

<120>  Procedimiento de obtención de la proteína IFNAR recombinante así como
su uso como antiviral

<130>  FIMABIS-15008

<160>  7

<170>  PatentIn version 3.5

<210>  1
<211>  2479
<212>  DNA
<213>  Homo sapiens

<400>  1
agatgtaaaa gtcaagagaa gactctaaaa atagcaaaga tgcttttgag ccagaatgcc      60

ttcatcttca gatcacttaa tttggttctc atggtgtata tcagcctcgt gtttggtatt     120

tcatatgatt cgcctgatta cacagatgaa tcttgcactt tcaagatatc attgcgaaat     180

ttccggtcca tcttatcatg ggaattaaaa aaccactcca ttgtaccaac tcactataca     240

ttgctgtata caatcatgag taaaccagaa gatttgaagg tggttaagaa ctgtgcaaat     300

accacaagat cattttgtga cctcacagat gagtggagaa gcacacacga ggcctatgtc     360

accgtcctag aaggattcag cgggaacaca acgttgttca gttgctcaca caatttctgg     420

ctggccatag acatgtcttt tgaaccacca gagtttgaga ttgttggttt taccaaccac     480

attaatgtga tggtgaaatt tccatctatt gttgaggaag aattacagtt tgatttatct     540

ctcgtcattg aagaacagtc agagggaatt gttaagaagc ataaacccga aataaaagga     600

aacatgagtg gaaatttcac ctatatcatt gacaagttaa ttccaaacac gaactactgt     660

gtatctgttt atttagagca cagtgatgag caagcagtaa taaagtctcc cttaaaatgc     720

accctccttc cacctggcca ggaatcagaa ttttcataac tttttagcct ggccatttcc     780

taacctgcca ccgttggaag ccatggatat ggtggaggtc atttacatca acagaaagaa     840

gaaagtgtgg gattataatt atgatgatga aagtgatagc gatactgagg cagcgcccag     900

gacaagtggc ggtggctata ccatgcatgg actgactgtc aggcctctgg gtcaggcctc     960

tgccacctct acagaatccc agttgataga cccggagtcc gaggaggagc ctgacctgcc    1020

tgaggttgat gtggagctcc ccacgatgcc aaaggacagc cctcagcagt tggaactctt    1080

gagtgggccc tgtgagagga gaaagagtcc actccaggac ccttttcccg aagaggacta    1140

cagctccacg gaggggtctg ggggcagaat taccttcaat gtggacttaa actctgtgtt    1200

tttgagagtt cttgatgacg aggacagtga cgacttagaa gcccctctga tgctatcgtc    1260

tcatctggaa gagatggttg acccagagga tcctgataat gtgcaatcaa accatttgct    1320

ggccagcggg gaagggacac agccaacctt tcccagcccc tcttcagagg gcctgtggtc 1380

cgaagatgct ccatctgatc aaagtgacac ttctgagtca gatgttgacc ttggggatgg 1440

ttatataatg agatgactcc aaaactattg aatgaacttg gacagacaag cacctacagg 1500

gttctttgtc tctgcatcct aacttgctgc cttatcgtct gcaagtgttc tccaagggaa 1560

ggaggaggaa actgtggtgt tcctttcttc caggtgacat cacctatgca cattcccagt 1620

atggggacca tagtatcatt cagtgcattg tttacatatt caaagtggtg cactttgaag 1680

gaagcacatg tgcacctttc ctttacacta atgcacttag gatgtttctg catcatgtct 1740

accagggagc agggttcccc acagtttcag aggtggtcca ggaccctatg atatttctct 1800

tctttcgttc tttttttttt ttttttgaga cagagtctcg ttctgtcgcc caagctggag 1860

cgcaatggtg tgatcttggc tcactgcaac atccgcctcc cgggttcagg tgattctcct 1920

gcctcagcct ccctcgcaag tagctgggat tacaggcgcc tgccaccatg cctagcaaat 1980

ttttgtattt ttagtggaga caggatttta ccatgttggc caggctggtc tcgaactcct 2040

gacctcaagt gatctgccct cctcagcctc gtaaagtgct gggattacag gggtgagccg 2100

ctgtgcctgg ctggccctgt gatatttctg tgaaataaat tgggccaggg tgggagcagg 2160

gaaagaaaag gaaaatagta gcaagagctg caaagcaggc aggaagggag gaggagagcc 2220

aggtgagcag tggagagaag gggggccctg cacaaggaaa cagggaagag ccatcgaagt 2280

ttcagtcggt gagccttggg cacctcaccc atgtcacatc ctgtctcctg caattggaat 2340

tccaccttgt ccagccctcc ccagttaaag tggggaagac agactttagg atcacgtgtg 2400

tgactaatac agaaaggaaa catggcgtcg ggagagggga taaaacctga atgccatatt 2460

ttaagttaaa aaaaaaaaa 2479

<210> 2
<211> 239
<212> PRT
<213> Homo sapiens

<400> 2

Met Leu Leu Ser Gln Asn Ala Phe Ile Phe Arg Ser Leu Asn Leu Val
1               5                   10                  15

Leu Met Val Tyr Ile Ser Leu Val Phe Gly Ile Ser Tyr Asp Ser Pro
            20                  25                  30

Asp Tyr Thr Asp Glu Ser Cys Thr Phe Lys Ile Ser Leu Arg Asn Phe
        35                  40                  45

Arg Ser Ile Leu Ser Trp Glu Leu Lys Asn His Ser Ile Val Pro Thr
    50                  55                  60

His Tyr Thr Leu Leu Tyr Thr Ile Met Ser Lys Pro Glu Asp Leu Lys
65                  70                  75                  80

Val Val Lys Asn Cys Ala Asn Thr Thr Arg Ser Phe Cys Asp Leu Thr
                85                  90                  95

Asp Glu Trp Arg Ser Thr His Glu Ala Tyr Val Thr Val Leu Glu Gly
            100                 105                 110

Phe Ser Gly Asn Thr Thr Leu Phe Ser Cys Ser His Asn Phe Trp Leu
        115                 120                 125

Ala Ile Asp Met Ser Phe Glu Pro Pro Glu Phe Glu Ile Val Gly Phe
    130                 135                 140

Thr Asn His Ile Asn Val Met Val Lys Phe Pro Ser Ile Val Glu Glu
145                 150                 155                 160

Glu Leu Gln Phe Asp Leu Ser Leu Val Ile Glu Glu Gln Ser Glu Gly
                165                 170                 175

Ile Val Lys Lys His Lys Pro Glu Ile Lys Gly Asn Met Ser Gly Asn
            180                 185                 190

Phe Thr Tyr Ile Ile Asp Lys Leu Ile Pro Asn Thr Asn Tyr Cys Val
        195                 200                 205

Ser Val Tyr Leu Glu His Ser Asp Glu Gln Ala Val Ile Lys Ser Pro
    210                 215                 220

Leu Lys Cys Thr Leu Leu Pro Pro Gly Gln Glu Ser Glu Phe Ser
225                 230                 235

<210>  3
<211>  46
<212>  DNA
<213>  Artificial Sequence

<220>
<223>  Sentido 5'-3'

<400>  3
taaggcctct gtcgacattt catatgattc gcctgattac acgatg                46

<210>  4
<211>  45
<212>  DNA
<213>  Artificial Sequence

<220>
<223>  Antisentido 5'-3'

<400>  4
cagaattcgc aagctttgaa aattctgatt cctggccagg tggaa                    45


<210>  5
<211>  932
<212>  DNA
<213>  Artificial Sequence

<220>
<223>  PLASMIDO con el inserto

<400>  5
cggcgtagag gatcgaggtc tcgatcccgc gaaattaata cgactcacta taggggaatt      60

gtgagcggat aacaattccc ctcttgaaat aattttgttt aactttaaga aggagatata     120

ccatgggtaa ggcctctgtc gacatttcat atgattcgcc tgattacaca gatgaatctt     180

gcactttcaa gatatcattg cgaaatttcc ggtccatctt atcatgggaa ttaaaaaacc     240

actccattgt accaactcac tatacattgc tgtatacaat catgagtaaa ccagaagatt     300

tgaaggtggt taagaactgt gcaaatacca caagatcatt ttgtgacctc acagatgagt     360

ggagaagcac acacgaggcc tatgtcaccg tcctagaagg attcagcggg aacacaacgt     420

tgttcagttg ctcacacaat ttctggctgg ccatagacat gtcttttgaa ccaccagagt     480

ttgagattgt tggttttacc aaccacatta atgtgatggt gaaatttcca tctattgttg     540

aggaagaatt acagtttgat ttatctctcg tcattgaaga acagtcagag ggaattgtta     600

agaagcataa acccgaaata aaaggaaaca tgagtggaaa tttcacctat atcattgaca     660

agttaattcc aaacacgaac tactgtgtat ctgtttattt agagcacagt gatgagcaag     720

cagtaataaa gtctccctta aaatgcaccc tccttccacc tggccaggaa tcagaatttt     780

caagcttgcg aattctggcg gccgcctggt tccgcgtggc tctccgggcg ctgcaggtca     840

taatcataat cataatcata atcataatca caattaatta attaatctag aggaagctga     900

gttggctgct gccaccgctg agcaataact ag                                   932


<210>  6
<211>  800
<212>  DNA
<213>  Artificial Sequence

<220>
<223>  Elisa_T7Up


<220>
<221>  misc_feature
<222>  (1)..(17)
<223>  n is a, c, g, or t

<220>
<221> misc_feature
<222> (25)..(28)
<223> n is a, c, g, or t

<220>
<221> misc_feature
<222> (74)..(80)
<223> n is a, c, g, or t

<220>
<221> misc_feature
<222> (85)..(85)
<223> n is a, c, g, or t

<220>
<221> misc_feature
<222> (799)..(800)
<223> n is a, c, g, or t

<400> 6
nnnnnnnnnn nnnnnnntat agggnnnngt gagcggataa caattcccct cttgaaataa 60

ttttgtttaa cttnnnnnnn gaganatacc atgggtaagg cctctgtcga catttcatat 120

gattcgcctg attacacaga tgaatcttgc actttcaaga tatcattgcg aaatttccgg 180

tccatcttat catgggaatt aaaaaaccac tccattgtac caactcacta tacattgctg 240

tatacaatca tgagtaaacc agaagatttg aaggtggtta agaactgtgc aaataccaca 300

agatcatttt gtgacctcac agatgagtgg agaagcacac acgaggccta tgtcaccgtc 360

ctagaaggat tcagcgggaa cacaacgttg ttcagttgct cacacaattt ctggctggcc 420

atagacatgt cttttgaacc accagagttt gagattgttg gttttaccaa ccacattaat 480

gtgatggtga aatttccatc tattgttgag gaagaattac agtttgattt atctctcgtc 540

attgaagaac agtcagaggg aattgttaag aagcataaac ccgaaataaa aggaaacatg 600

agtggaaatt tcacctatat cattgacaag ttaattccaa acacgaacta ctgtgtatct 660

gtttatttag agcacagtga tgagcaagca gtaataaagt ctcccttaaa atgcaccctc 720

cttccacctg gccaggaatc agaattttca aagcttgcga attctggcgg ccgcctggtt 780

ccgcgtggct ctccgggcnn 800

<210> 7
<211> 800
<212> DNA
<213> Artificial Sequence

<220>
<223> Elisa_T7Term

<220>
<221> misc_feature
<222> (1)..(1)
<223> n is a, c, g, or t

<220>
<221>  misc_feature
<222>  (3)..(3)
<223>  n is a, c, g, or t

<220>
<221>  misc_feature
<222>  (9)..(11)
<223>  n is a, c, g, or t

<220>
<221>  misc_feature
<222>  (14)..(14)
<223>  n is a, c, g, or t

<220>
<221>  misc_feature
<222>  (31)..(31)
<223>  n is a, c, g, or t

<220>
<221>  misc_feature
<222>  (33)..(36)
<223>  n is a, c, g, or t

<220>
<221>  misc_feature
<222>  (47)..(47)
<223>  n is a, c, g, or t

<220>
<221>  misc_feature
<222>  (61)..(65)
<223>  n is a, c, g, or t

<220>
<221>  misc_feature
<222>  (68)..(68)
<223>  n is a, c, g, or t

<220>
<221>  misc_feature
<222>  (70)..(70)
<223>  n is a, c, g, or t

<220>
<221>  misc_feature
<222>  (72)..(72)
<223>  n is a, c, g, or t

<220>
<221>  misc_feature
<222>  (77)..(81)
<223>  n is a, c, g, or t

<220>
<221>  misc_feature
<222>  (108)..(108)
<223>  n is a, c, g, or t

<220>
<221>  misc_feature

<222>  (390)..(391)
<223>  n is a, c, g, or t

<220>
<221>  misc_feature
<222>  (477)..(478)
<223>  n is a, c, g, or t

<220>
<221>  misc_feature
<222>  (719)..(719)
<223>  n is a, c, g, or t

<220>
<221>  misc_feature
<222>  (721)..(723)
<223>  n is a, c, g, or t

<220>
<221>  misc_feature
<222>  (725)..(729)
<223>  n is a, c, g, or t

<220>
<221>  misc_feature
<222>  (778)..(800)
<223>  n is a, c, g, or t

<400>  7
ntntaagann ngantatacc catgggtaag ncnnnngtcg acatttncat atgatttcgc      60

nnnnnttncn cngatgnnnn ntgcactttc aagatatcat tgcgaaantt tccggtccat     120

cttatcatgg gaattaaaaa accactccat tgtaccaact cactatacat tgctgtatac     180

aatcatgagt aaaccagaag atttgaaggt ggttaagaac tgtgcaaata ccacaagatc     240

attttgtgac ctcacagatg agtggagaag cacacacgag gcctatgtca ccgtcctaga     300

aggattcagc gggaacacaa cgttgttcag ttgctcacac aatttctggc tggccataga     360

catgtctttt gaaccaccag agtttgagan ngttggtttt accaaccaca ttaatgtgat     420

ggtgaaattt ccatctattg ttgaggaaga attacagttt gatttatctc tcgtcannga     480

agaacagtca gagggaattg ttaagaagca taaacccgaa ataaaaggaa acatgagtgg     540

aaatttcacc tatatcattg acaagttaat tccaaacacg aactactgtg tatctgttta     600

tttagagcac agtgatgagc aagcagtaat aaagtctccc ttaaaatgca ccctccttcc     660

acctggccag gaatcagaat tttcaaagct tgcgaattct ggcggccgcc tggttccgng     720

nnncnnnnng ggcgctgcag gtcataatca taatcataat cataatcata atcacaannn     780

nnnnnnnnnn nnnnnnnnnn                                                 800